# FASCICULE DE BREVET EUROPEEN

(11) **EP 1 751 100 B1**
(45) Date de publication et mention de la délivrance du brevet: **09.01.2008**
(21) Numéro de dépôt: 05770861.2
(22) Date de dépôt: 13.05.2005
(51) Int. Cl.: C07D 207/333, C07D 207/335, C07D 207/32, A61K 31/4015

(54) **DERIVES ARYLPYRROLIQUES, ARYLFULRANIQUES ET ARYLTHIOPHENIQUES A ACTION ANTITUBULINE, PROCEDE DE PREPARATION ET UTILISATION EN TANT QU"ANTIMITOTIQUE**
ARYLPYRROL MIT ANTITUBULINWIRKUNG, ARYLFULRAN- UND ARYLTHIOPENDERIVATE, VERFAHREN ZUR HERSTELLUNG UND VERWENDUNG ALS ANTIMITOTIKUM
ANTI-TUBULIN ACTING ARYLPYRROL, ARYLFULRAN AND ARYLTHIOPENE DERIVATIVES, METHOD FOR THE PREPARATION AND USE THEREOF AS AN ANTIMITOTIC

(30) Priorité: 14.05.2004 FR 0405278
(43) Date de publication de la demande: 14.02.2007
(73) Titulaire: Centre National de la Recherche Scientifique (CNRS), 75016 Paris (FR); UNIVERSITE DE RENNES I, F-35000 Rennes (FR)
(72) Inventeur: ARLOT, Yannick, F-35235 Thorigne-Fouillard (FR); MARTIN, Bénédicte, 35000 RENNES (FR); DELCROS, Jean-Guy, 35700 Rennes (FR); ALCARAZ, Gilles, 31500 Toulouse (FR); PAULUS, Olivier, F-91220 Bretigny S/Orge (FR)
(74) Mandataire: Ahner, Francis
(86) Numéro de dépôt international: PCT/FR2005/001207
(87) Numéro de publication internationale: WO 2005/115979

(56) Documents cités:
- WO-A-20/04029040
- US-B1- 6 258 841
- SHUJI A ET AL: "Lipase-catalyzed domino kinetic resolution/intramolecular Diels-Alder reaction: one-pot synthesis of optically active 7-oxabicyclo[2.2.1]heptenes from furfuryl alcohols and beta-substituted acrylic acids" CHEM. EUR. J., vol. 8, no. 18, 2002, pages 4255-4264, XP002312205
- KIN-FAI C ET AL: "Diastereoselective aldol reactions of furaldehyde using a chiral boronate as auxiliary: application to the synthesis of enantiomerically pure and highly functionalized 2,3-disubstituted furanly alcohols" EUR. J. ORG. CHEM., vol. 1, 2003, pages 82-91, XP002312206
- KIN-FAI C ET AL: "Diastereoselective addition reactions of furyl aldehydes using chirl boronates as auxiliary: application to the enantioselective synthesis of 2,3-disubstituted furyl alcohols" ORGANIC LETTERS, vol. 3, no. 25, 2001, pages 3991-3994, XP002312208
- LI Q ET AL: "Discovery and development of antimitotic agents that inhibit tubulin polymerisation for the treatment of cancer" EXPERT. OPIN. THER. PATENTS, vol. 12, no. 11, 2002, pages 1663-1702, XP002312209

## Description

La présente invention concerne de nouveaux dérivés arylpyrroliques arylfuraniques et arylthiophénique ayant une action antitubuline, avantageusement antimitotique.

Il existe dans les compartiments cellulaires, une série de fibres qui contribuent à la forme et la texture de la cellule : il s'agit du cytosquelette dont la composition majoritaire est assurée par la présence de micro filaments d'actine, de microtubules et de filaments intermédiaires le tout associé à des complexes de protéines.

Le réseau de microtubules joue un rôle essentiel dans la morphologie, l'endocytose, l'exocytose et le cycle cellulaire. La dynamique des microtubules en relation avec le guanosine tri phosphate (GTP) est indispensable pour la formation du fuseau mitotique, les microtubules sont par voie de conséquence des éléments clés de la ségrégation des chromosomes pendant la méiose et également la mitose ; ils participent ainsi au maintien de la stabilité génétique.

D'un point de vue organisation, les microtubules sont des structures cylindriques qui résultent de l'assemblage longitudinal hélicoïdal de 13 proto filaments lesquels se forment par l'association de molécules de tubuline à partir d'un ou plusieurs centres de nucléation présents dans la cellule (microtubule organising center = MTOC). Ces centres organisateurs sont les centrioles situés dans le cytoplasme. La majorité des microtubules sont le siège d'un cycle rapide de polymérisation dépolymérisation selon un modèle d'instabilité dynamique. La tubuline constituant les microtubules est une protéine globulaire hétérodimérique constituée de deux chaines polypeptidiques de tubuline α et tubuline β. Ces hétérodimères sont alignés en alternance αβ pour former des protofilaments. Les hétérodimères sont polarisés et par voie de conséquence les protofilaments et les microtubules ont une polarité intrinsèque. Certains réseaux de microtubules ont des propriétés très différentes en ce qui concerne leur stabilité, leur géométrie ainsi que leur résistance vis à vis de certaines drogues antimitotiques. Les fuseaux de division sont également des structures labiles capables de s'assembler et se désassembler rapidement. Une grande hétérogénéité des microfilaments est concentrée dans la partie C terminale des différentes iso formes de la tubuline elle même impliquée dans l'association avec des MAPs (Microtubule associated proteins).

Les MAPs ont un rôle médiateur entre les composants de la cellule et les microtubules. Elles influent sur la structure dynamique des microtubules et co purifient avec la tubuline. On distingue deux types de MAPs ; les MAPs structurales (i.e . MAP2, la protéine tau, Stable Tubule Only Polypeptide : STOP) et les MAPs motrices à activité enzymatique dont les kinésines et la dynéine. Une double fonctionnalité peut être assurée par une même famille de MAPs.

Pendant la division cellulaire, les microtubules formant le fuseau mitotique, fonctionnent dans le dessein de ségréguer les chromosomes dupliqués afin de les orienter dans le plan de clivage. La dynamique du fuseau mitotique permet donc l'accrochage des centromères sur les microtubules du fuseau et après séparation des chromatides soeurs, la migration des chromosomes à chaque pôle du fuseau mitotique. Cette étape est un des évènements responsable de la transmission de l'information génétique d'une cellule mère à la cellule fille. Les aberrations chromosomiques sont l'une des caractéristiques principales des cellules cancéreuses, marquant ainsi l'importance du maintien d'une information génétique parfaitement stable. Certaines de ces anomalies de formation ou destruction du fuseau pourraient avoir un rôle dans la transformation cellulaire, par la perte d'un gène régulateur négatif de la croissance, celle d'un gène responsable de l'intégrité du génome ou par l'amplification, la surexpression ou l'activation par mutation d'un oncogène.

Une tumeur se développe au sein d'un organe lorsque certaines de ses cellules ont perdu leur faculté d'inhibition de contact et se divisent indéfiniment. Une des stratégies utilisée dans le traitement des cancers consistera à bloquer la division cellulaire en ciblant la machinerie nécessaire à la ségrégation des chromosomes lors de la mitose.

De par sa diversité fonctionnelle, la tubuline et les microtubules sont des cibles de choix pour le traitement des cancers ou autres maladies liées à des modifications de structure ou d'organisation de cette molécule telles que les maladies auto immunes, les maladies hyper-prolifératives et les maladies neurodégénératives. D'autre part, des agents anti-mitotiques agissant sur la tubuline (Tubulin binding agents) ou les protéines associées induisent des dommages vasculaires et inhibent les mitoses dans les tumeurs.

Des molécules telles que le Taxol, la vinblastine et la colchicine sont des composés anti-mitotiques utilisés en thérapie anti-cancéreuse. La colchicine diminue la polymérisation des microtubules. Le taxol stabilise les microtubules par interaction sur deux sites spécifiques sur la tubuline β. Il est un composant antimitotique préférentiel par son action stabilisante des microtubules.

Des composés arylfuraniques sont décrits dans Chem. Eur. J (2002, 8, 18 4255), Eur.J. Org. Chem. (2003,82-31), Organic Letters (2001, 3, 25, 3991-94). US 6,258,841 décrit des composés agissant sur la tubiline.

Les inventeurs ont découvert de façon surprenante que de nouveaux composés baptisés paulusines faisant partie de la famille des composés arylpyrroliques, - furaniques ou bien -thiophéniques sont capables d'agir sur la mise en place du fuseau mitotique et de contrôler son intégrité fonctionnelle. Les paulusines sont des composés originaux synthétisés pour répondre aux problèmes d'instabilité génétique liés aux anomalies de formation du fuseau.

La présente invention concerne donc des composés de formule générale I suivante 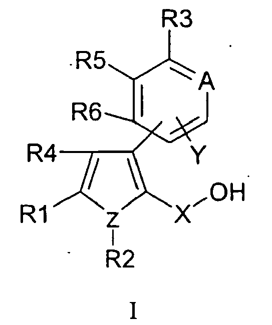 dans laquelle :
Z représente N, O ou S
R1, R3, R4, R5 et R6 représentent indépendamment l'un de l'autre un atome d'hydrogène, un atome de fluor, un groupe alkyle en C₁-C₁₂ linéaire ou ramifié, cycloalkyle en C₃-C₁₂, alcényle en C₂-C₁₂ linéaire ou ramifié, CH₂-B ou CH₂-CH₂-B dans lesquels B représente un groupe phényle substitué ou non par un ou plusieurs groupes choisis parmi un groupe alkyle en C₁-C₆ linéaire ou ramifié, alkoxy en C₁-C₆ linéaire ou ramifié, NH₂, NO₂, CN, COOH, CO₂(alkyle) en C₁-C₆ linéaire ou ramifié, CONH₂, CONH(alkyle) en C₁-C₆ linéaire ou ramifié, CON(alkyle)₂ en C₁-C₆ linéaire ou ramifié Cl, Br, I, OH, COCF₃, OSO₂CF₃ ; naphtyle; anthracényle; 9H-fluorényle substitué ou non en position 9 par un ou deux groupe alkyle en C₁-C₁₂ linéaire ou ramifié ; anisyle ou pyridinyle,
R2 représente H, un groupe alkyle en C₁-C₁₂ linéaire ou ramifié, phényle, benzyle, CO₂alkyle en C₁-C₆ linéaire ou ramifié, CO₂benzyle, CO₂alcényle en C₂-C₆ linéaire ou ramifié, tosyle, mésyle, 9-fluorenylmethoxycarbonyl (Fmoc), NH₂ ou NH(alkyle) en C₁-C₆ linéaire ou ramifié, N(alkyle)₂ en C₁-C₆ linéaire ou ramifié ou NH tertiobutyloxycarbonyl ou NHCO₂CH₂ phényle ou R2 est absent quand Z représente O.
X représente un groupe alkyle en C₁-C₁₂ linéaire ou ramifié, hydroxyalkyle en C₁-C₁₂ linéaire ou ramifié, ou aminoalkyle en C₁-C₁₂ linéaire ou ramifié.
A représente un groupe CH, un atome d'azote ou un groupe NL⁺ dans lequel L représente un groupe alkyle en C₁-C₁₂ linéaire ou ramifié.
Y représente un atome d'hydrogène, un groupe alkyle en C₁-C₁₂, linéaire ou ramifié, cycloalkyle en C₃-C₁₂, OH, CN, N₃, alcoxy en C₁-C₁₂ linéaire ou ramifié, hydroxyalkyle en C₁-C₁₂ linéaire ou ramifié, aminoalkyle en C₁-C₁₂ linéaire ou ramifié, N₂⁺, NZ1-NHZ2, NH-NZ1Z2 ou NZ1Z2 dans lesquels
Z1 et Z2 représentent indépendamment l'un de l'autre un atome d'hydrogène ; un groupe alkyle en C₁-C₁₂ linéaire ou ramifié ; cycloalkyle en C₃-C₁₂ ; phényle substitué ou non par un ou plusieurs groupes choisis parmi un groupe alkyle en C₁-C₆ linéaire ou ramifié, alkoxy en C₁-C₆ linéaire ou ramifié, NH₂, CN, COOH, CO₂(alkyle) en C₁-C₆ linéaire ou ramifié, CONH₂, CONH(alkyle) en C₁-C₆ linéaire ou ramifié, CON(alkyle)₂ en C₁-C₆ linéaire ou ramifié Cl, Br, I, OH, COCF₃ ou OSO₂CF₃ ; benzyle; anisyle; pyridinyle ; C(O)-W ; C(S)-W ou C(NH)-W, dans lesquels
W représente un groupe alkyle en C₁-C₁₂ linéaire ou ramifié, cycloalkyle en C₃-C₁₂, alcoxy en C₁-C₁₂ linéaire ou ramifié, alkylthio en C₁-C₁₂ linéaire ou ramifié ou NQQ1,
   dans lesquels
   Q et Q1 représentent indépendamment l'un de l'autre un atome d'hydrogène, un groupe alkyle en C₁-C₁₂ linéaire ou ramifié, cycloalkyle en C₃-C₁₂, ou CH(M)CO₂Ml dans lesquels
   M et M1 représentent indépendamment l'un de l'autre un atome d'hydrogène, un groupe alkyle en C₁-C₁₂ linéaire ou ramifié, cycloalkyle en C₃-C₁₂, alcényle en C₂-C₁₂ linéraire ou ramifié, phényle, benzyle, CH₂-B ou CH₂-CH₂-B dans lesquels B est tel que défini ci-dessus.

Dans un mode de réalisation de la présente invention, le composé selon la présente invention est tel que Z représente N.
Avantageusement, A représente le groupe CH.
De façon avantageuse, Y représente un groupe NH₂ ou un atome d'hydrogène.
Avantageusement, X représente un groupe alkyle en C₁-C₁₂ linéaire ou ramifié, avantageusement un groupe propyle.

Dans un mode de réalisation particulier de l'invention les composés selon la présente invention sont choisis dans le groupe constitué par 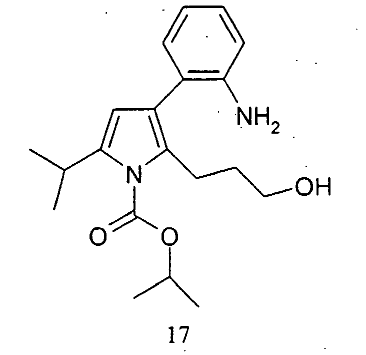 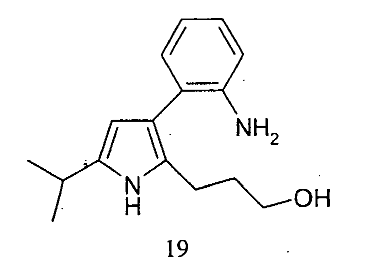 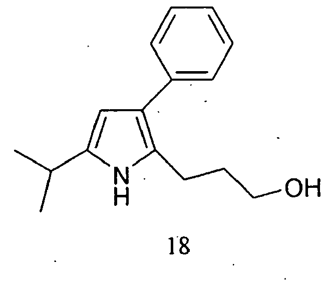 et

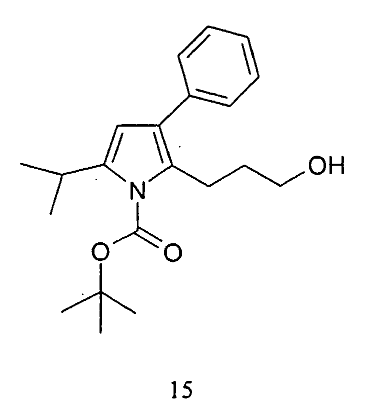

La présente invention concerne également un procédé de préparation des composés comprenant les étapes de :
a)synthèse d'un motif pyrrolylalkylcarbinole, furanylalkylcarbinole ou thiophénylalkylcarbinole de formule générale II suivante 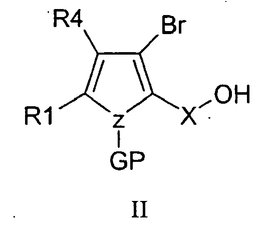 dans laquelle Z, X, R₁ et R4 sont tels que définis ci-dessus dans la formule générale 1 et GP représente un groupe protecteur de l'azote lorsque Z représente N, ou est absent lorsque Z représente O ou S,
b) fonctionnalisation du motif pyrrolylalkylcarbynol furanylalkylcarbinole ou thiophénylalkylcarbinole en introduisant un motif arylique ou hétéroarylique en position 3 du cycle pyrole, furane ou thiophéne.

Avantageusement, dans le cas où X représente (CH₂)₃, l'étape a) consiste en la cyclodéshydratation d'aminocétones β-γ insaturées de formule III suivante : 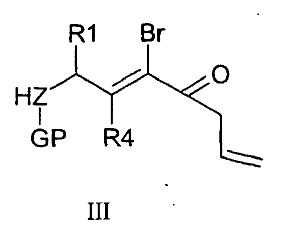 dans laquelle Z, R1, R4 et GP sont tels que définis ci-dessus dans la formule générale II afin d'obtenir le produit de formule IV suivante 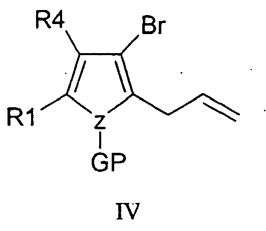 dans laquelle Z, R1, R4 et GP sont tels que définis ci-dessus dans la formule générale II, suivi de l'introduction de la fonction alcool par hydroboration/oxydation du produit de formule IV afin d'obtenir le produit de formule II.

De façon avantageuse, dans le cas où R2, R3, R4, R5 et R6 représentent un atome d'hydrogène, Y représente un groupe NH₂, A représente un groupe CH, X représente (CH₂)₃ et Z représente N, l'étape b) consiste en :
- un couplage mixte pallado-catalysée de type Suzuki-Miyaura du composé de formule II, dans lequel R4 représente un atome d'hydrogène, X représente (CH₂)₃, Z représente N et GP représente un groupe protecteur de l'azote, et l'acide 2-triazène boronique de façon à obtenir un composé de formule V suivante : 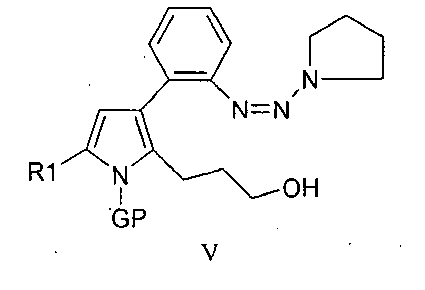
dans laquelle GP représente un groupe protecteur de l'azote et R1 est tel que défini ci-dessus dans la formule générale I,
- une déprotection de la fonction triazène afin d'obtenir le groupe de formule VI suivant 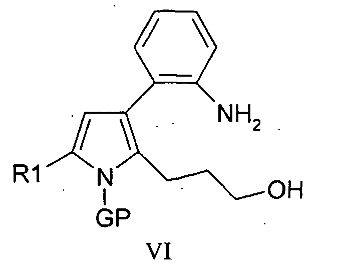
dans laquelle GP représente un groupe protecteur de l'azote et R1 est tel que défini ci-dessus dans la formule générale I
- une déprotection spécifique du groupement protecteur GP afin d'obtenir le composé de formule I dans laquelle R2, R3, R4, R5 et R6 représentent un atome d'hydrogène, Y représente un groupe NH₂, A représente un groupe CH, X représente (CH₂)₃, Z représente N et R1 est tel que défini ci-dessus dans la formule générale I.

La méthodologie de synthèse utilisée est connue de l'homme du métier et a été adaptée à partir d'un procédé décrit par Paulus et al (Eur. J. Org. Chem. 2002, 2565-2572).
La synthèse de paulusines à motif pyrrole va être plus particulièrement décrite. Bien entendu, de nombreuses variations synthétiques sont possibles permettant d'élaborer un grand nombre de composés hétérocycliques apparentés.
La voie de synthèse se décompose donc en deux temps forts :
- le 1^{er} consiste en la synthèse d'un motif pyrrolylalkylcarbinol fonctionnalisable sous la forme d'un 3-bromopyrrole N-protégé possédant une chaîne latérale alcool qui est portée par le carbone en position α de l'azote du cycle.
   Le motif pyrrolalkylcarbinol à la formule générale suivante VII 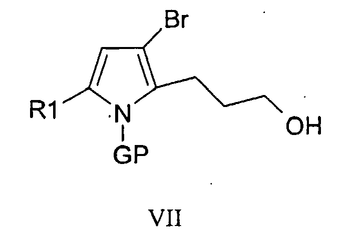 dans laquelle
   GP représente un groupe protecteur de l'atome d'azote et
   R1 est tel que défini dans la formule générale I.

   Le noyau pyrrole polyfonctionnalisé est obtenu par cyclodéshydratation d'aminocétones β-γ insaturées (étape v) avec des rendements de 60 à 90%. Ces aminocétones β-γ insaturées sont le plus souvent obtenues en plusieurs étapes (i-iv,i) à partir de β-aminoalcools N-protégés commerciaux. De cette manière, on obtient tout d'abord un 3-bromopyrrole N-protégé possédant une chaîne latérale insaturée.
   La fonction alcool est ensuite introduite dans ce cas *via* une séquence classique d'hydroboration/oxydation (étape vi) en milieu basique. 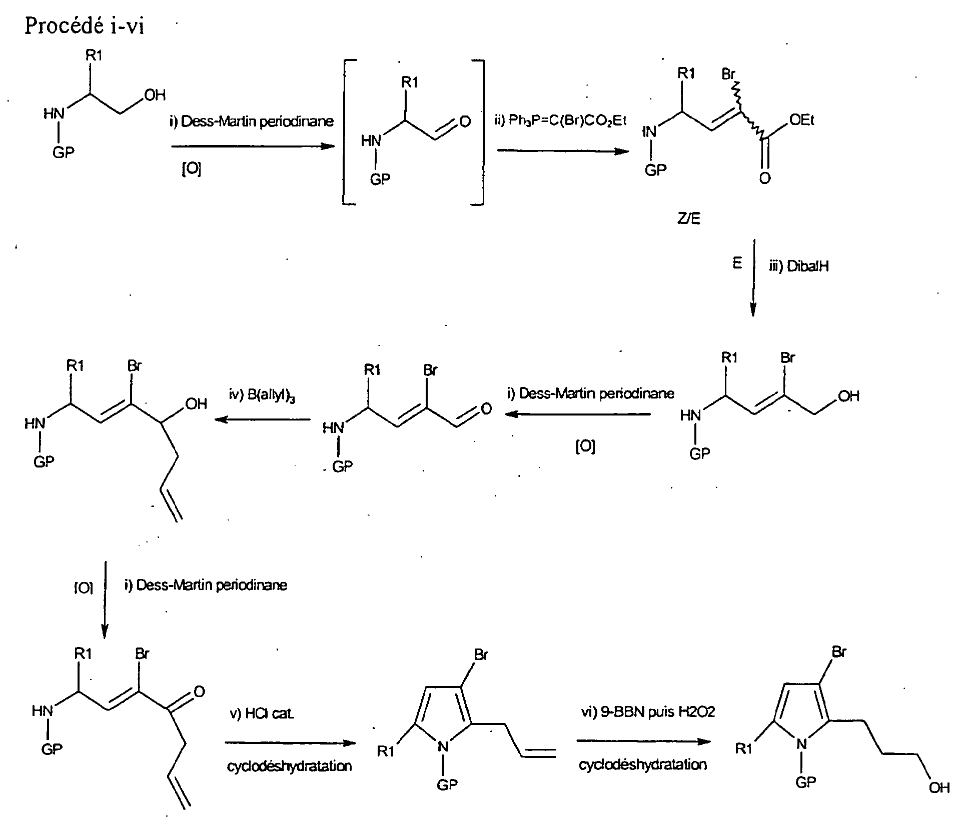
- le 2^{ème} temps fort consiste à fonctionnaliser le motif pyrrolylalkylcarbinol en introduisant le motif arylique ou hétéroarylique en position 3 du cycle pyrrole. Dans l'exemple ci-après, un motif aniline est introduit par réaction de couplage mixte pallado-catalysée de type Suzuki-Miyaura. L'aniline est introduite sous une forme protégée originale, celle d'un phényltriazène, à partir de l'acide 2-triazène boronique (étape *vii*) réactif également original. Une étape de déprotection de la fonction triazène permet *in fine* de régénérer la fonction amine du noyau aniline (étape *viii*)*.* Puis, une étape de déprotection spécifique du groupement protecteur (GP) utilisé peut alors être mise en oeuvre si nécessaire pour obtenir l'anilinopyrrolylalcool N-déprotégé correspondant.

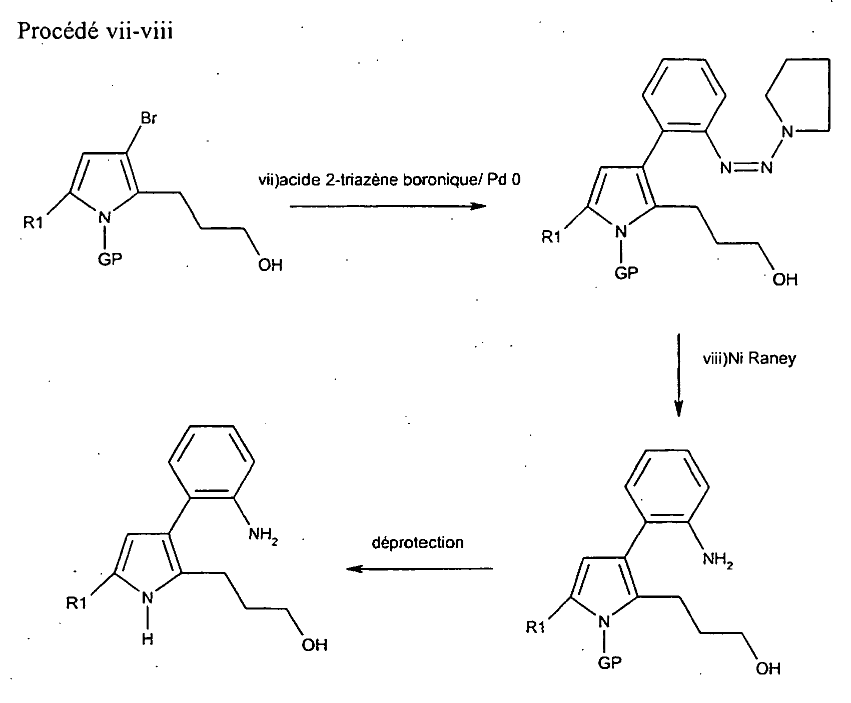

Bien entendu, cette voie de synthèse ne se restreint pas à la seule introduction d'un motif anilino.

La présente invention concerne également une composition pharmaceutique comprenant un composé selon la présente invention et un excipient pharmaceutiquement acceptable, un composé pour son utilisation en tant que médicament, avantageusement antimitotique, de façon avantageuse antitumoral.

Ce médicament peut également être destiné au traitement des maladies auto-immunes, des maladies hyper prolifératives et des maladies neurodégénératives.

La présente invention concerne également un composé selon la présente invention pour son utilisation en tant que médicament destiné au traitement ou à la prévention du cancer.

La présente invention concerne donc des compositions pharmaceutiques comprenant à titre de principe actif un des composés définis ci-dessus et un excipient approprié. Ces compositions peuvent être formulées pour l'administration aux mammifères, y compris l'homme. La posologie varie selon le traitement et selon l'affection en cause. Ces compositions sont réalisées de façon à pouvoir être administrées par la voie digestive ou parentérale.
Dans les compositions pharmaceutiques de la présente invention pour l'administration orale, sublinguale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux ou aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intràoculaire et les formes d'administration rectale.
Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.
On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.
Les poudres ou granules dispersibles dans l'eau peuvent contenir l'ingrédient actif en mélange avec des agents de dispersion ou des agents mouillants, ou des agents de mise en suspension, de même qu'avec des correcteurs de goût ou des édulcorants.
Pour une administration rectale, on recourt à des suppositoires qui sont préparés avec des linats fondant à la température rectale, par exemple du beurre de cacao ou des polyéthylèneglycols.
Pour une administration parentérale, intranasale ou intraoculaire, on utilise des suspensions aqueuses, des solutions salines isotoniques ou des solutions stériles et injectables qui contiennent des agents de dispersion et/ou des agents mouillants phamacologiquement compatibles.
Le principe actif peut être formulé également sous forme de microcapsules, éventuellement avec un ou plusieurs supports additifs.

La présente invention concerne en outre des composés de formule générale V suivante: 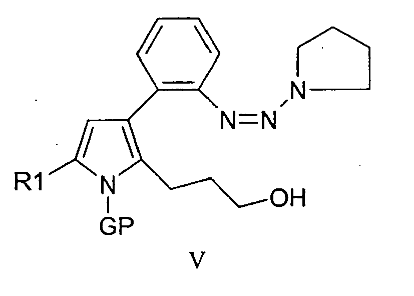 dans lequel GP représente un groupe protecteur de l'azote et R1 représente un atome d'hydrogène, un atome de fluor, un groupe alkyle en C₁-C₁₂ linéaire ou ramifié, cycloalkyle en C₃-C₁₂, alcényle en C₂-C₁₂ linéaire ou ramifié, CH₂-B ou CH₂-CH₂-B
dans lesquels B représente un groupe phényle substitué ou non par un ou plusieurs groupes choisis parmi un groupe alkyle en C₁-C₆ linéaire ou ramifié, alkoxy en C₁-C₆ linéaire ou ramifié, NH₂, NO₂, CN, COOH, CO₂(alkyle) en C₁-C₆ linéaire ou ramifié, CONH₂, CONH(alkyle) en C₁-C₆ linéaire ou ramifié, CON(alkyle)₂ en C₁-C₆ linéaire ou ramifié Cl, Br, I, OH, COCF₃, OSO₂CF₃ ; naphtyle ; anthracényle ; 9H-fluorényle substitué ou non en position 9 par un ou deux groupe alkyle en C₁-C₁₂ linéaire ou ramifié ; anisyle ou pyridinyle.

Elle concerne en outre un composé de formule générale VI suivante : 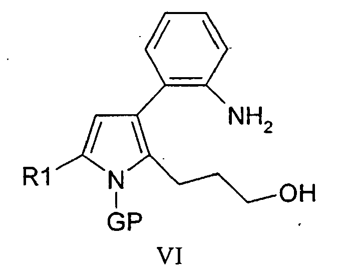 dans lequel GP représente un groupe protecteur de l'azote et R1 représente un atome d'hydrogène, un atome de fluor, un groupe alkyle en C₁-C₁₂ linéaire ou ramifié, cycloalkyle en C₃-C₁₂, alcényle en C₂-C₁₂ linéaire ou ramifié, CH₂-B ou CH₂-CH₂-B
dans lesquels B représente un groupe phényle substitué ou non par un ou plusieurs groupes choisis parmi un groupe alkyle en C₁-C₆ linéaire ou ramifié, alkoxy en C₁-C₆ linéaire ou ramifié, NH₂, NO₂, CN, COOH, CO₂(alkyle) en C₁-C₆ linéaire ou ramifié, CONH₂, CONH(alkyle) en C₁-C₆ linéaire ou ramifié, CON(alkyle)₂ en C₁-C₆ linéaire ou ramifié Cl, Br, I, OH, COCF₃, OSO₂CF₃ ; naphtyle ; anthracényle ; 9H-fluorényle substitué ou non en position 9 par un ou deux groupe alkyle en C₁-C₁₂ linéaire ou ramifié ; anisyle ou pyridinyle.

Les exemples suivants sont donnés à titre indicatif non limitatif

### I. Exemple 1 : préparation des composés 17, 18, 19

Le schéma générale de synthèse est le suivant :

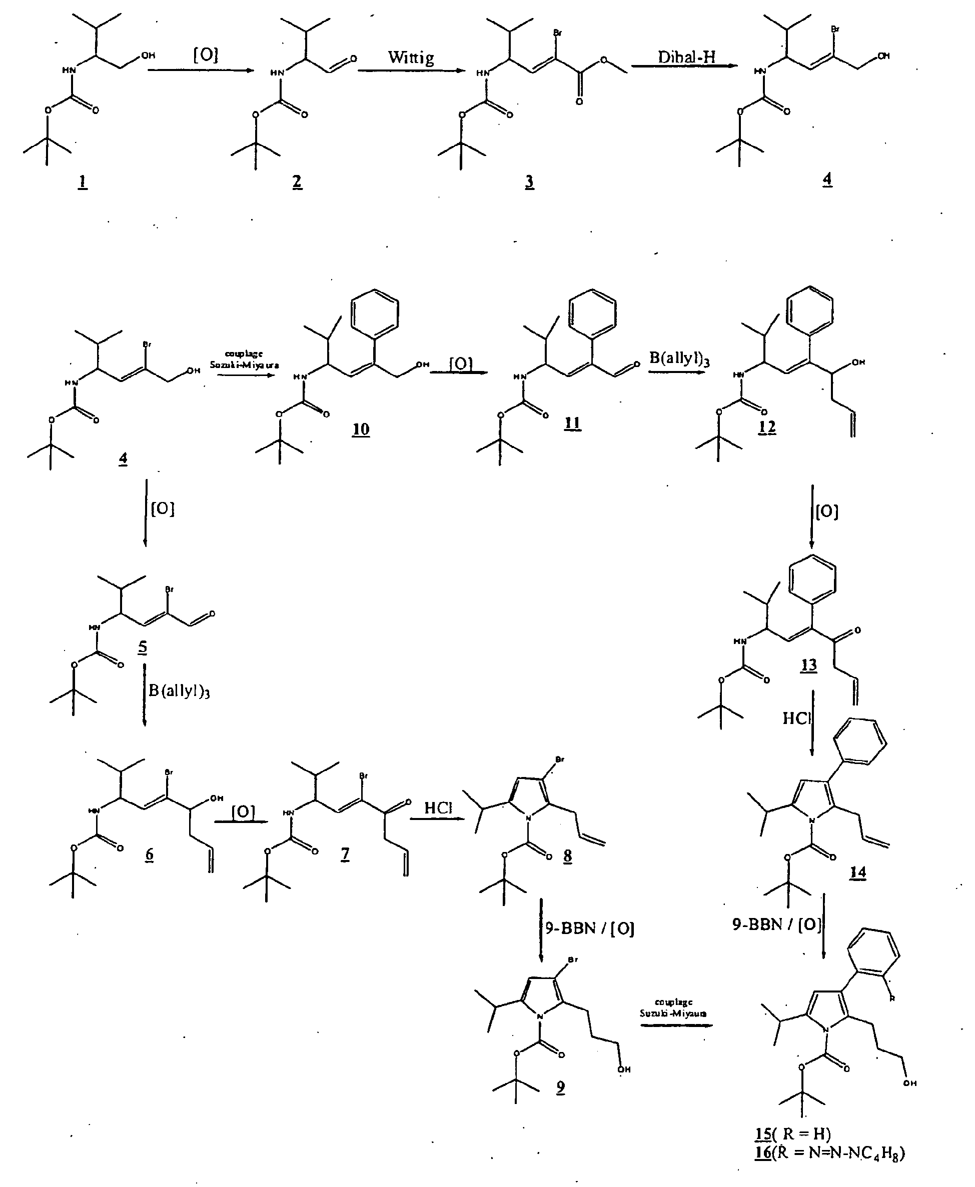

Le couplage Susuki-Miyaura permettant d'obtenir le composé 16 à partir du composé 9 correspond au schéma réactionnel suivant :

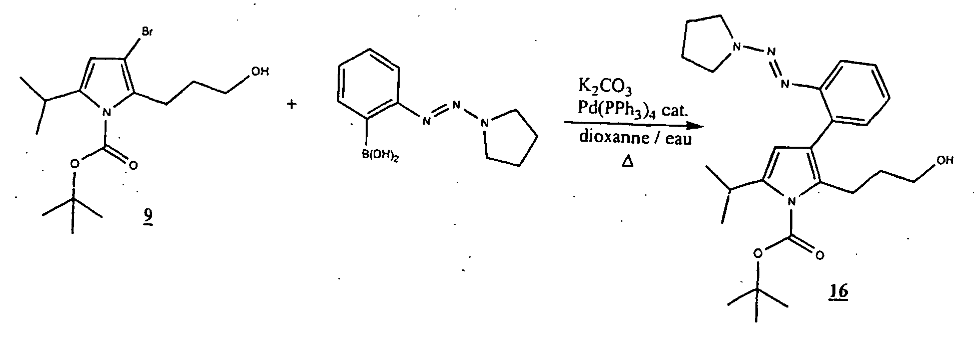

De même le passage du composé 8 au composé 9 se fait selon la réaction suivante:

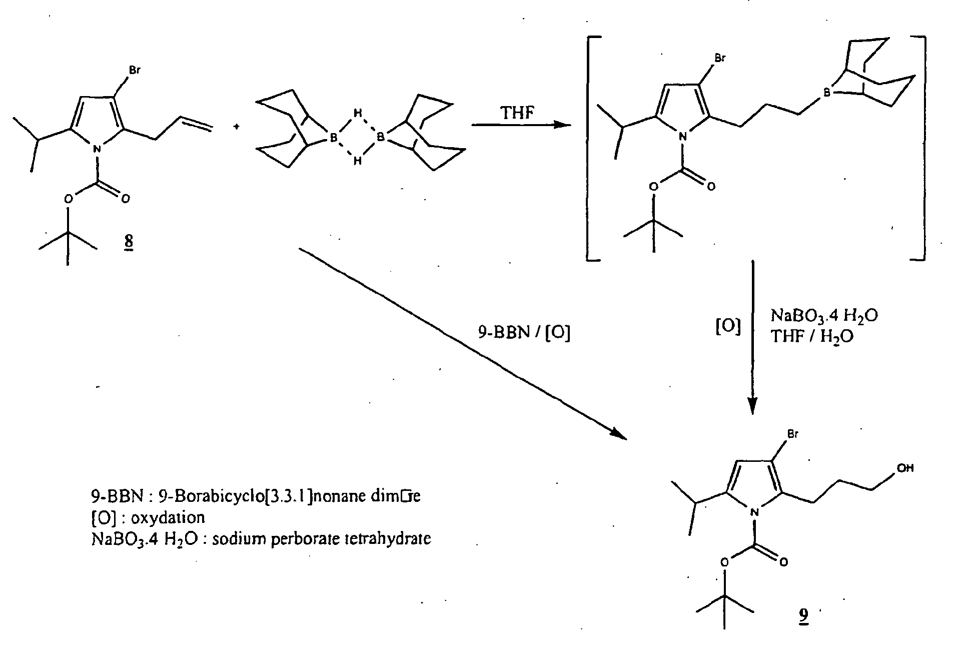

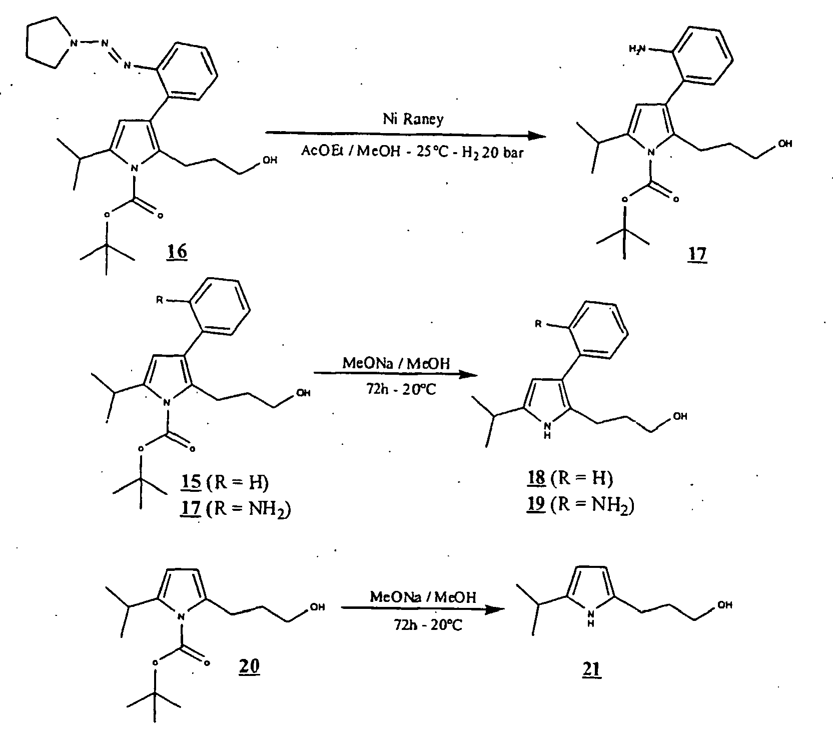

Pour les composés **1** et **2**, le procédé utilisé est celui décrit dans l'article de Paulus et al. (Eur. J. Org. Chem. 2002, 2565-2572).

### (S)-N-(tert-Butoxycarbonyl)valinal 2

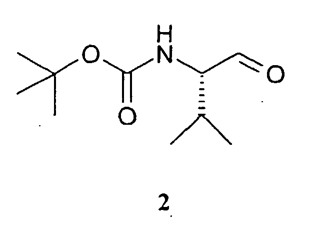

il s'agit d'une huile jaune pâle
Rf= 0,45 (AcOEt/Hept 1/1)
**RMN (200 MHz, CDCl₃) ¹H** : δ
0,87 et 0,96. d, 6 H, ³J = 7.0 Hz, CH₃)
1,38 (s, 9 H, (CH₃)₃)
2,20 (m, 1 H, C*H*(CH₃)₂)
4,15 (m, 1 H; CH)
5,12 (d large, 1 H, NH)
9,55 (s, 1H, CHO)
**RMN (50 MHz, CDCl₃) ¹³C :**δ
17,9 et 19,4 (CH₃)
28,6 [(CH₃)₃]
29,4 [CH(CH₃)₂]
65,0 (CH)
80,2 [*C*(CH₃)₃]
156,2 (NCO)
200,8 (CHO)

### (2Z)-2-bromo-4-[N-(tert-butoxycarbonyl)amino]-5-méthylhexènoate de méthyle(Z-3) et son isomère (E-3)

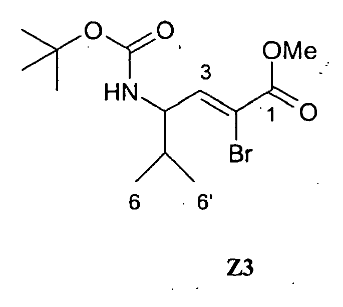

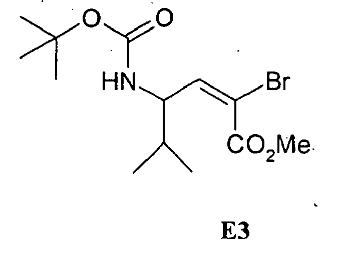

A une solution, refroidie à -20,0°C, de (méthoxycarbonylméthylène)triphénylphosphorane (Ph₃P=CH-CO₂Me, 7,90 g, 23,5 mmol) dans le dichlorométhane (60 ml), sont successivement ajoutés avec un intervalle de 20 minutes; de la N-Bromosuccinimide (4,60 g ,25,9 mmol) et du carbonate de potassium anhydre (8,10 g, 58,8 mmol). Le mélange, jaune-brun, est laissé sous agitation 20' et du N-(Boc)Valinal 2 (4,74 g, 23,5 mmol) fraîchement préparé, dissous dans le CH₂Cl₂ (20 ml) est instillé. L'addition terminée, le mélange est abandonné 30 min à -20,0°C et 1 h à la température ambiante. Un contrôle CCM indique la disparition totale de l'aldéhyde. Le mélange est filtré sur fritté recouvert de célite, et le fritté est rincé à l'éther. Après évaporation sous vide du filtrat, l'huile jaune obtenue est chromatographiée sur gel de silice (gradient d'élution Heptane/AcOEt 90/10→75/25). 2,10 g (27%, Rf = 0,46 (AcOEt/Hept 1/3)) d'isomère E pur, 0,63 g du mélange (7%, Z+E) et 3,2 g (41%, Rf= 0,35 (AcOEt/Hept 1/3)) d'isomère Z pur sont obtenus.
Rendement = 75 %
Le composé Z-3 est une huile incolore
**RMN ¹H (200 MHz, CDCl₃) :** δ 1.00 (d, 6 H, ³J = 6.8 Hz, H₆ et H_{6'}), 1.48 (s, 9 H, (CH₃)₃), 1.97 (m, 1 H, H₅), 3.88 (s, 3 H, OMe), 4.42 (s large, 1 H, H₄), 4.69 (s large, 1 H, NH), 7.17 (d, 1 H, ³J = 6.8 Hz, H₃)

**RMN ¹³C (50 MHz, CDCl₃) :** δ 17.2 et 17.8(C6 et C6'), 27.3 [(CH₃)₃], 31.1 (C5), 52.4 (OCH₃), 56.2 (C4), 78.8 [C(CH₃)₃], 115.4 (C2), 144.2 (C3), 154.3 (NC=O), 161.8(C1)

Le composé ***E*-3** est une huile incolore
**RMN ¹H (200 MHz, CDCl₃)** : δ 0.78 (d, ³J = 6.8 Hz, H₆ et H₆,), 1.26 (s, 9 H, (CH₃)₃), 1.72 (m, 1 H, H₅),3.67 (s, 3 H, OMe), 4.53 (m, 2 H, NH + H₄), 6.32 (d, 1 H,³J = 7.0 Hz, H₃)
**RMN ¹³C (50 MHz, CDCl₃)** : δ 18.5 et 19.4 (C6 et C6'), 28.7 [(CH₃)₃], 32.9 (C5), 53.5 (OCH₃), 56.1 (C4), 80.0 [C(CH₃)₃], 113.0 (C2), 148.2 (C3), 156.7 (NC=O), 163.4 (C1) **Analyse élémentaire** (mélange E+Z): calc % C 46,44 % H 6,60 % N 4,17 mes % C 46,66 % H 6,81 % N 4,05

### (2Z)-2-bromo-4-[N-(tert-butoxycarbonyl)amino]-5-méthylhex-2-èn-1-ol 4 C₁₂H₂₂BrNO₃ M = 308 g.mol-1

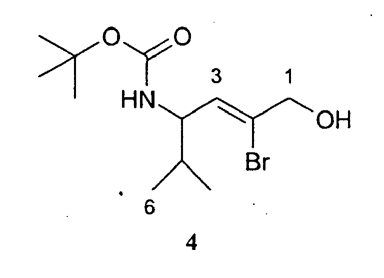

A une solution d'ester **Z-3** (2,60 g, 7,7 mmol), dans le dichlorométhane (50 ml), refroidie à -78°C, sont instillés sur un intervalle de 15 minutes 990 µL (1,0 éq) de BF₃.OEt fraîchement distillé, puis sur 1 heure, 23,4 ml (3,0 éq) de DIBAL (solution 1M/toluène). Le mélange réactionnel est maintenu 3 h à -78°C puis est neutralisé à - 50°C par addition lente de 5 ml d'acide acétique. A 0°C, 30 ml d'une solution aqueuse saturée en NH₄Cl sont additionnés. Le gel formé est cassé par ajout d'une solution aqueuse saturée en acide citrique en prenant soin de ne pas descendre à un pH<3-4. La phase aqueuse est extraite à l'AcOEt. Les phases organiques réunies sont lavées à la saumure et séchées sur MgSO₄. Après filtration et évaporation du solvant, l'huile jaune obtenue est purifiée sur gel de silice (AcOEt/Hept 1/2), pour conduire à une huile incolore qui, après trituration au pentane et séchage prolongé à la pompe à palettes, cristallise sous la forme d'un solide blanc (1,73 g, 5,6 mmol).
Rendement (Rdt) = 72 % Rf = 0,45 (AcOEt/Hept 1/1) Pf=74-76°C
**RMN ¹H (200 MHz, CDCl₃) :** δ 0.96 (d, 6 H, ³J = 6.8 Hz, H₆ et H₆,), 1.46 (s, 9 H, (CH₃)₃), 1.90 (s large, 1 H, H₅), 2.00 (s large, 1 H, OH), 4.27 (d, 2 H, ³J = 1.2 Hz, H₁), 4.30 (m, 1 H, H₄), 4.58 (s large, 1 H, NH), 5.98 (d, 1 H, ³J = 8.6 Hz, H₃)
**RMN ¹³C (50 MHz, CDCl₃) :** δ 18.6 et 19.1 (C6 et C6'), 28.8 [(CH₃)₃], 32.9 (C5), 57.3 (C4), 68.2 (C1), 79.9 [C(CH₃)₃], 128.5 (C2), 128.9 (C3), 156.5 (NC=O)

| | |
|---|---|
| **HRMS (IE):** | m/z calculé pour C₉H₁₅⁷⁹BrNO₃ ([M-C3H7]^{+•}) : 264,0235 |
| | Mesuré : 264,0242 |
| **Analyse élémentaire**/ | calc % C 46,76 % H 7,19 % N 4,54 |
| | mes % C 46,93 % H 7,35 % N 4,39 |

### (2Z)-2-bromo-4-[N-(tert-butoxycarbonyl)amino]-5-méthylhéx-2-énal 5

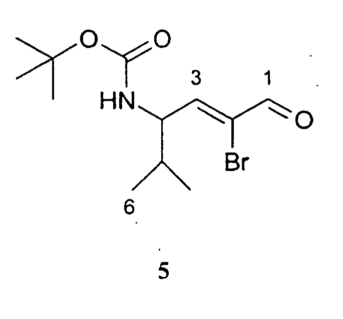

Le Dess-Martin Périodinane (630 mg, 1,3 éq) est ajouté en une fois à une solution d'alcool allylique **4** (347 mg, 1,1 mmol) dans le CH₂Cl₂ (15 ml). Le mélange réactionnel est agité 1 h à la température ambiante avant dilution par de l'éther et neutralisation par une solution aqueuse saturée en Na₂S₂O₃ (15 ml) et une solution aqueuse saturée en NaHCO₃ (15 ml). La phase aqueuse est extraite à l'éther puis les phases organiques réunies sont lavées à la saumure et séchées sur MgSO₄. Après filtration et évaporation du solvant, l'huile résiduelle obtenue est purifiée par filtration sur un tube d'alun recouvert de 2-3 cm de silice (éluant : Et₂O) pour conduire à l'énal **5** (324 mg, 1,06 mmol, propre en RMN ¹H) sous la forme d'une huile incolore.
Rdt = 95 %
Rf = 0,55 (AcOEt/Hept 1/1)
**RMN ¹H (300 MHz, CDCl₃) :** 0.96 et 0.98(d, 6 H, ³J = 6.8 Hz, H₆ et H_{6'}), 1.37 (s, 9 H, (CH₃)₃), 1.99 (s large, 1 H, H₅), 4.46 (m, 1H, H₄), 4.95 (s large, 1 H, NH), 6.99 (d, 1 H, ³J = 7.3 Hz, H₃), 9.17 (s, 1H, H₁)
**RMN ¹³C (75 MHz, CDCl₃) :** δ
18.3 et 19.0 (C6 et C6'), 28.4 [(CH₃)₃], 32.1 (C5), 57.2 (C4), 80.2 [C(CH₃)₃], 128.5 (C2), 153.8 (C3), 155.3 (NC=O), 186.1 (CHO)

| | | | | | |
|---|---|---|---|---|---|
| **HRMS (IE):** | m/z calculé pour C8H₁₂ ⁷⁹BrNO₃ ([M-C₄H₈^{•}]⁺) : | | | | 249,0000 |
| | Mesuré : | | | | 248,9997 |
| | m/z calculé pour C₉H₁₃⁷⁹BrNO₃ ([M-C₃H₇^{•}]⁺ : | | | | 262,0078 |
| | Mesuré : | | | | 262,0078 |
| **Analyse élémentaire:** | calc % C 47,07 % H 6,58 % N 4,57 | | | | |
| | mes % C 47,12 % H 6,65 % N 4,46 | | | | |

### 5-méthyl-4(N-tert-Butoxycarbonyl)amino-2-bromo-1-allyl-(2Z)-hexèn-1-ol 6

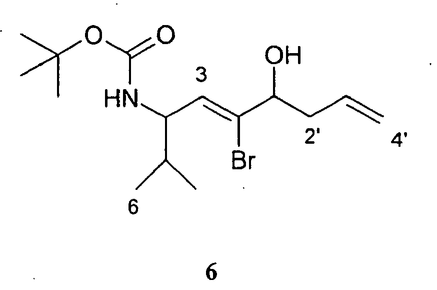

Le triallylborane (460 µl; 2.42 mmol) est lentement ajouté à une solution d'énal **5** (370 mg; 1,21 mmol) dans du tétrahydrofurane (THF) (10 ml) refroidie à -70°C. Après 1 h d'agitation, le mélange est laissé remonter en température et est neutralisé à 0°C avec une solution aqueuse saturée en NH₄Cl (10 ml). Après séparation des phases et extraction à l'AcOEt, les phases organiques réunies sont lavées à la saumure, séchées sur MgSO₄ et filtrées. L'huile résiduelle obtenue après évaporation du solvant, est purifiée sur gel de silice (AcOEt/Hept 1/2) pour conduire à l'alcool **6** (385 mg; 1,11 mmol) qui est obtenu sous la forme d'une huile incolore.
Rdt = 92 %
Rf=0,51 (AcOEt/ Hept 1/1)

*Mélange de deux diastéréoisomères D1 et D2 en proportion 50*/*50 d'après la RMN ¹H*.
**RMN** ¹H **(300 MHz, CDCl₃)** : δ 0.92 (m, 6 H, ³J = 6.8 Hz, H₆), 1.44 (s, 9 H, (CH₃)₃), 1.85 (s large, 1H, H₅), 2.38-2.53 (m, 3 H, H_{2'}+ OH), 4.16 et 4.19 (t, 1 H, ³J = 6.1 Hz, H₁), 4.30 (s large, 1 H, H₄), 4.59 (s large, 1 H, NH), 5.11 (d, 1 H, ³J = 9.1 Hz, H_{4'cis}), 5.15 (d, 1 H, ³J = 15.3 Hz, H_{4'trans}), 5.72 (m, 1 H, H_{3'}), 5.87 et 5.91 (d, 1 H, ³J = 8.7 Hz, H₃)
**RMN ¹³C (75 MHz, CDCl₃)** : δ 18.4 et 18.7 (C6 et C6'), 28.4 [(CH₃)₃], 32.5 (C5), 40.0 (C2'), 56.8 (C4), 75.5 (C1), 79.8 [*C*(CH₃)₃], 118.6(C4'), 129.0 et 129.7 (C3'), 131.5 et 131.9 (C2), 133.2 et 133.3, (C3), 155.3 (NC=O)

| | | | | | |
|---|---|---|---|---|---|
| **HRMS (IE):** | m/z calculé pour C₁₂H₁₉NO₃⁷⁹Br ([M-C₃H₇^{•}]⁺) : | | | | 304,0548 |
| | Mesuré : | | | | 304,0541 |
| **Analyse élémentaire:** | calc | % C 51,73 | % H 7,52 | % N 4,02 | |
| | mes | % C 51,92 | % H 7,63 | % N 4,05 | |

### (2Z)-5-méthyl-4(N-tert-Butoxycarbonyl)amino-2-bromo-1-allyhexèn-1-one 7

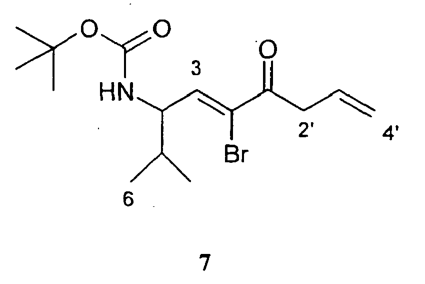

Le périodinane de Dess-Martin (640 mg; 1,51 mmol) est ajouté à une solution d'alcool **6** (350 mg; 1,00 mmol) dans le dichlorométhane (10 ml) et le mélange est abandonné jusqu'à disparition complète de l'alcool en CCM (1h). Le mélange est alors dilué avec de l'éther et évaporé sous vide. Le brut est ensuite repris avec un mélange AcOEt/Hept (3 ml; 1/3 v/v) puis filtré sur gel de silice (4-5 cm dans un tube d'alun; éluant AcOEt/Hept 1/3) pour conduire après collecte des fractions et évaporation du solvant à l'énone **7** (325 mg; 0,94 mmol) sous la forme d'une huile incolore.
Rdt = 94 %
Rf = 0,64 (AcOEt/ Hept 1/1)
**RMN ¹H (200 MHz, CDCl₃)** : δ 1.01 (d, 6 H, ³J = 6.8 Hz, H6), 1.47 (s, 9 H, (CH₃)₃), 1.99 (s large, 1H, H5), 3.62 (dm, 2 H, ³J = 6.6 Hz, H_{2'}), 4.44 (s large, 1 H, H₄), 4.78 (s large, 1 H, NH), 5.19 (dm, 1 H, ³J = 16.9 Hz, H_{4'trans}), 5.27 (dm, 1 H, ³J = 10.3 Hz, H_{4'cis}), 5.98 (ddt, 1 H, ³J= 17.1 Hz, ³J = 10.4 Hz, ³J = 6.6 Hz, H_{3'}), 7.02 (d, 1 H, ³J = 8.3 Hz, H₃)
**RMN ¹³C (50 MHz, CDCl₃)** : δ 18.6 (C6), 119.6 (C4'), 19.3 (C6'), 126.4 (C2), 28.8 [(CH₃)₃], 130.8 (C3') 32.5 (C5), 144.2 (C3), 44.1 (C2'), 155.7 (NC=O), 58.1 (C4), 192.7 (CHO), 80.4 [C(CH₃)₃]
**HRMS (IE):** m/z calculé pour C₁₂H₁₇NO₃⁷⁹Br ([M-C₃H₇^{•}]⁺) : 302,0392
Mesuré : 302,0380

### 1-(tert-Butoxycarbonyl)-2-isopropyl-4-bromo-5-allylpyrrole 8

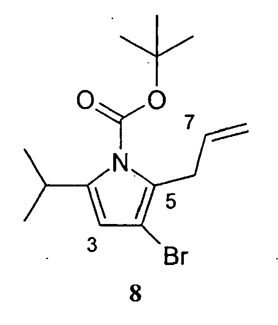

Une solution d'énone **7** (310 mg; 0,90 mmol) dans le dichlorométhane (10 ml) est acidifiée par de l'HCl (450 µl, solution 2 M dans Et₂O) et le mélange est agité à la température ambiante. L'avancement de la réaction est suivi en CCM et après disparition du produit de départ le mélange réactionnel est neutralisé avec une solution aqueuse saturée en NaHCO₃ (10 ml). Après séparation des phases et extraction de la phase aqueuse à l'AcOEt, les phases organiques sont réunies, lavées à la saumure, séchées sur MgSO₄ puis filtrées. L'huile obtenue après évaporation du solvant est purifiée sur gel de silice (AcOEt/Hept 1/6) pour conduire au pyrrole **8** (195 mg; 0,59 mmol) sous la forme d'une huile incolore.
Rdt = 66 %
Rf= 0,73 (AcOEt/ Hept 1/3)
**RMN ¹H (300 MHz, CDCl₃)** : δ 1.19 (d, 6 H, ³J = 6.8 Hz, CH₃), 1.59 (s, 9 H, (CH₃)₃), 3.42 (app quint, 1H, ³J = 6.8 Hz, C*H*(CH₃)₂), 3.61 (dm, 2 H, ³J =5.6 Hz, H₆), 4.92 (dm, 1H, ³J = 17.3 Hz, H₈ₜᵣₐₙₛ), 5.03 (dm, 1H, ³J =10.3 Hz, H_{8cis}), 5.89 (ddt, 1H, ³J = 17.3, 10.3 et 5.6 Hz, H₇), 5.98 (d, 1 H, ⁴J = 0.9 Hz, H₃)
**RMN ¹³C (75 MHz, CDCl₃)** : δ 23.1 (CH₃), 27.1 [*C*H(CH₃)₂], 28.1 [(CH₃)₃], 31.2 (C6), 84.5 [*C*(CH₃)₃], 101.1 (C4), 109.7 (C3), 115.4 (C8), 129.2 (C5), 135.4 (C7), 142.8 (C2), 149.5 (NC=0)
**HRMS (IE):** m/z calculé pour C₁₅H₂₂N⁷⁹BrO₂ ([M]^{+•}) : 327,0834 Mesuré : 327,0837

### N-(tert-Butoxycarbonyl)-2-isopropyl-4-bromo-5-(3-hydroxypropyl)pyrrole 9

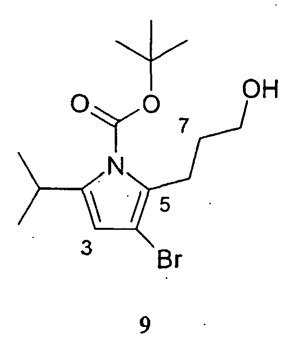

Sous atmosphère d'argon, une solution de 9-BBN (216 mg; 1,77 mmol) dans le THF (3 ml), refroidie à -20°C est lentement ajoutée à une solution de pyrrole **8** (195 mg; 0,59 mmol) dans le THF (2 ml). Le mélange est abandonné 30 minutes à -20°C et 20 h sous agitation à la température ambiante. 1 ml d'EtOH (16,9 mmol), 420 µl de soude 5N (2,1 mmol) et 720 µL d'eau oxygénée à 35% (en masse dans l'eau / 1 ml = 9,33 mmol / 6,71 mmol) sont alors successivement ajoutés. Le mélange se porte instantanément au reflux. Après 30 minutes, 10 ml de saumure sont ajoutés. Le mélange est extrait à l'AcOEt. Les phases organiques sont séchées sur MgSO₄ puis concentrées sous vide. L'huile jaune obtenue est purifiée sur gel de silice (AcOEt/Hept 1/3) pour conduire à l'alcool **9** (98 mg; 0,28 mmol) sous la forme d'une huile incolore.
Rdt = 48 %
Rf = 0,21 (AcOEt/ Hept 1/3)
**RMN ¹H (200 MHz, CDCl₃) :** δ 1.21 (d, 6 H, ³J = 6.8 Hz, CH³), 1.64 (s, 9 H, (CH₃)₃), 1.84 (quint, 3 H, ³J = 7.3 Hz, H₇ + OH), 2.96 (t, 2 H, ³J = 7.1 Hz, H₆), 3.41 (app quint, 1H, ³J = 6.8 Hz, C*H*(CH₃)₂), 3.66 (t, 2 H, ³J = 6.3 Hz, H₈), 5.98 (d, 1H, ⁴J = 1.0 Hz, H₃)
**RMN ¹³C (50 MHz, CDCl₃)** : δ 23.4 (CH₃), 85.1 [C(CH₃)₃], 23.7 (C6), 101.0 (C4), 27.6 [CH(CH₃)₂], 110.1 (C3), 28.3 [(CH₃)₃], 132.0 (C5), 32.8 (C7), 142.8 (C2), 62.3 (C8), 150.1 (NC=O)
**HRMS (IE):** m/z calculé pour C₁₅H₂₄⁷⁹BrNO₃ ([M]^{+•}) : 345,0939
Mesuré : 345,0923

### (2E),5-méthyl-4-(N-tert-butoxycarbonyl)amino-2-phényl-2-hexèn-1-ol 10

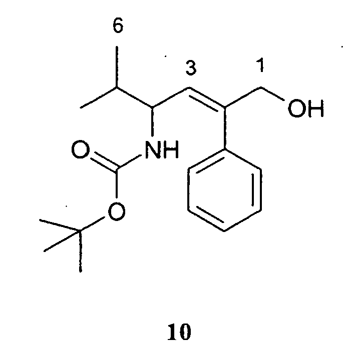

Un mélange dioxane/H2O (dégazé) (10 ml, 4/1 V/V) contenant l'alcool bromé **4** (770 mg ; 2,5 mmol), l'acide phénylboronique (335 mg ;1,1 éq), du carbonate de potassium (690 mg; 2,0 éq.) et du Pd(Ph₃)₄ (90 mg ; 0,03 éq.) est porté au reflux pendant 8 h sous argon. De retour à la température ambiante le mélange est filtré sur tube d'alun rempli d'un lit de quelques centimètres de célite. Au filtrat est ajouté une solution aqueuse saturée en NaCl (10 ml). Après séparation des phases et extraction de la phase aqueuse à l'AcOEt, les phases organiques sont lavées avec de la saumure et séchées sur MgSO₄ avant d'être concentrées sous vide. L'huile obtenue est purifiée sur gel de silice (AcOEt/Hept 1/2) pour conduire à l'alcool **10** (690 mg ; 2,3 mmol) sous la forme d'une mousse jaune pâle.
Rdt = 90 %
Rf= 0,38 (AcOEt/Hept 1/1)
**RMN¹H (200 MHz, CDCl₃) :** δ 0.83 (d, 6 H, ³J = 6.8 Hz, H₆), 1.44 (s large, 9 H, (CH₃)₃), 1.70 (m, 1 H, H₅), 2.23 (m, 1 H, OH), 4.00 (m, 1 H, H₄), 4.27 (dd, 2 H, ³J = 7.7 Hz , ⁴J = 1.2 Hz, H₁), 4.55 (d large, 1 H, ³J = 7.7 Hz, NH), 6.62 (dt, 1 H, ³J = 9.7 Hz, ⁴J = 1.4 Hz, H₃), 7.25-7.43 (m, 5 H, Har)

*Note :* 2 *rotamères sont détectés à la température ambiante en ¹³C*
**RMN ¹³C (75 MHz, CDCl₃)** : δ 18.3 (C6), 18.5 (C6'), 28.3[(CH₃)₃], 33.2,33.4 (C5), 54.0, 55.0 (C4), 66.9 (C1), 78.8, 79.3 [C(CH₃)₃], 125.3 (C3), 127.2 (CHar), 128.2 (CHar), 128.4 (CHar), 138.0 (^{IV}Car), 142.7 (C2), 155.1, 155.9 (NC=O)
**HRMS (IE)**: m/z calculé pour C₁₅H₂₀NO₃ ([M-C₃H₇^{•}]⁺): 262,1443 Mesuré : 262,1457
m/z calculé pour C₁₄H₁₉NO₃ ([M-C₄H₈]^{+•}):249,1365
Mesuré : 249,1383

### 5-méthyl-4-(N-tert-butoxycarbonyl)amino-2-phényl-(2E)-hexén-1-al 11

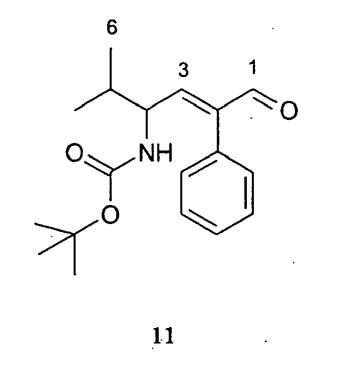

A une solution d'alcool allylique **10** (690 mg ; 2,26 mmol) dans le CH₂Cl₂ (30 ml) est ajouté le périodinane de Dess-Martin (1.44 g ; 1,5 éq). Après 30 minutes d'agitation à la température ambiante, de l'éther (50 ml) et une solution saturée en NaHCO₃ (30 ml) contenant Na₂S₂O₃ (3,0 g) sont ajoutés. Le mélange réactionnel est agité jusqu'à dissolution du précipité. Après séparation des phases, et extraction à l'éther, les phases organiques sont lavées à la saumure, séchées sur MgSO₄, et filtrées. L'huile résiduelle obtenue après évaporation du solvant est purifiée par passage sur un tube d'alun recouvert de 3-5 cm de silice (AcOEt/Hept 1/2). L'énal **11** (615 mg, 2,03 mmol, propre en RMN ¹H) est obtenu sous la forme d'une mousse incolore.
Rdt = 90 %
Rf= 0,56 (AcOEt / Hept 1/1)
**RMN¹H (300 MHz, CDCl₃) :** δ 0.84 (d, 3 H, ³J = 6.8 Hz, H₆), 0.87 (d, 3 H, ³J = 6.8 Hz, H_{6'}), 1.43 (s, 9 H, (CH₃)₃), 1.79 (sept, 1 H , ³J = 6.8 Hz, H₅), 4.32 (app. q, 1 H, ³J = 8.1 Hz, H₄), 4.63 (d, 1 H, ³J = 8.8 Hz, NH), 6.50 (d, 1 H, ³J = 9.1 Hz, H₃), 7.26-7.45 (m, 5 H, Har), 9.64 (s, 1 H, CHO)
**RMN ¹³C (75 MHz, CDCl₃) :** δ 18.2 (C6), 18.8 (C6'), 28.3 [(CH₃)₃], 32.7 (C5), 54.6 (C4), 79.5 [C(CH₃)₃], 128.1 (CHar), 128.3 (CHar), 129.3 (CHar), 132.1 (IVCar), 144.2 (C2), 153.5 (C3), 155.0 (NCO), 193.7 (CHO)
**HRMS (IE):** m/z calculé pour C₁₈H₂₅NO₃ ([M-C₃H₇^{•}]⁺): 260,1287 Mesuré : 260,1277

### (2E)-1-allyl-2-phényl-4-[N-(tert-butoxycarbonyl)amino]-5-méthylhex-2-èn-1-ol 12

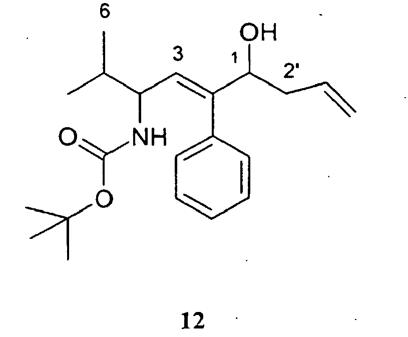

Mode opératoire identique à la préparation du composé **6**.

L'énal **11** (330 mg ; 1,09 mmol) conduit, après purification sur gel de silice (AcOEt/Hept 1/2) à l'alcool allylique **12** (312 mg ; 0,90 mmol) sous la forme d'une huile incolore.
Rdt = 83 %
Rf= 0,53 (AcOEt / Hept 1/1)
*Note : Le mélange est composé de 2 alcools diastéréoisomères en proportion 50*/*50 d'après la RMN ¹H.*
**RMN ¹H (300 MHz, CDCl₃) :** δ 0.75 et 0.82 (d, 6 H, ³J = 7.0 Hz, H₆), 1.37 et 1.41 (s large, 9 H, (CH₃)₃), 1.65 (m, 1 H, H₅), 1.83 (s large, 1 H, OH), 2.13 et 2.30 (m, 2 H, H_{2'}), 3.82 (m large, H₄), 4.31 et 4.39 (t(m), 1 H, ³J = 5.8 Hz, H1), 4.42 (s large, 1 H, NH), 5.06-5.14 (m, 2 H, H_{4'}), 5.57 et 5.59 (dd, 1 H, ³J = 9.4, ⁴J = 1.1 Hz, H₃), 5.78 (m, 1 H, H_{3'}), 7.19 (t large, 2 H, ³J = 4.2 Hz, Har), 7.29-7.37 (m, 3 H, Har)
**RMN ¹³C (75 MHz, CDCl₃) :** δ 18.4,18.5 et 18.8 (C6 et C6'), 28.4 ([(CH))₃]), 33.1 et 33.9 (C5), 40.0 (C2'), 54.1 et 55.3 (C4), 74.2 et 75.1 (C1), 78.8 et 79.3 [C(CH₃)₃], 118.1 et 118.4 (C4'), 127.2 et 127.3 (CHar), 128.2 et 128.3 (CHar), 129.0 et 129.1 (CHar), 134.3 et 134.5 (C3), 137.6 et 137.8 (IVCar), 144.9 et 145.0 (C2), 155.0 (NC=O)
**HRMS (IE):** m/z calculé pour C₁₈H₂₄NO₃ ([M-C₃H₇^{•}]⁺): 302,1756
Mesuré : 302,1752

| | | | | |
|---|---|---|---|---|
| **Analyse élémentaire** : | calc | % C 73,01 | % H 9,04 | % N 4,05 |
| | mes | % C 72,55 | % H 9,27 | % N 3,66 |

### (1-isopropyl-4-oxo-3-phenyl-hepta-2,6-dienyl)-carbamic acid tert-butyl ester 13.

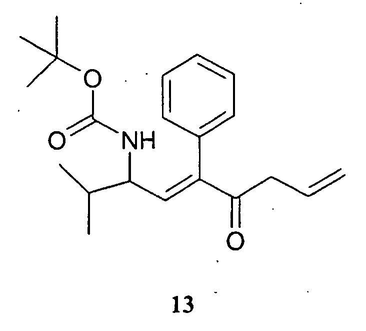

Le composé **13** est préparé de la même manière que le composé **5**. Il est obtenu avec un rendement de 68,8% après purification par chromatographie sur colonne de gel de silice (AcOEt / heptane : 1 / 2 en volume - Rf 0,66).
**RMN ¹H (CDCl₃, 300 MHz) : δ** 0.73 et 0.76 (d, ³J_{HH}=6.90 Hz, 6H, CH₃ iPr), 1.37 (s a, 9H, tBu), 1.64 (hept, ³J_{HH}=6.90 Hz, 1H, C*H* iPr), 3.22 (d large, 2H, CO-C*H*₂-CH=CH₂), 3.92 (m, 1H, N-C*H*), 4.73 (s large, 1H, N*H*), 4.96 (d large, 1H, CH₂-CH=C*H*ₐH_{b}), 5.02 (d large, 1H, CH₂-CH=CHₐ*H*_{b}), 5.87 (ddt, 1H, CH₂-C*H*=CHₐH_{b}), 6.64 (d, ³J_{HH}=9.64 Hz,1H, NH-CHiPr-C*H*), 7.10-7.40 (m, 5H, Ph)
**RMN ¹³C (CDCl₃, 75.47 MHz)** : δ 18.24 et 18.85 (2C, *C*H₃ iPr), 28.32 (3C, CH₃, tBu), 32.83 (1C, *C*H iPr), 44.66 (1C, *C*H₂-CH=CH₂), 54.59 (1C, NH-*C*H), 78.97 (1C, C^{IV}, tBu), 118.25 (1C, CH=*C*H₂), 127.76 (1C, p-*C*H Ph), 128.32 y 129.57 (1C, o-*C*H et m-CH Ph), 131.12 (1C,*C*H=C^{IV}), 135.27 (1C, C^{IV} Ph), 141.14 (1C, *C*H=CH₂), 142.34 (1C, CH=*C*^{IV}), 154.90 (1C, NH-C=O), 198.82 (1C, CH₂-*C*=O).
**HRMS (IE) :** m/z C₂₁H₂₉NO₃ ([M-C₄H₈^{•}]⁺) théo 287,15214, mesuré 287,1532 (3 ppm)

### 2-Allyl-5-isopropyl-3-phenyl-pyrrole-1-carboxylic acid tert-butyl ester 14.

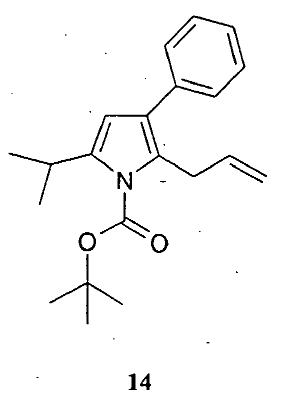

Le composé **14** est préparé de la même manière que le composé **8.** Il est isolé avec un rendement de 82,5% après purification par chromatographie sur colonne de gel de silice (AcOEt / heptane 1 /8 en volume - Rf 0,66).
**RMN ¹H (CDCl₃, 300 MHz) :** δ 1.33 (d, ³J_{HH}=6.8 Hz, 6H, CH₃), 1.67 (s, 9H, tBu), 3.55 (hept, ³J_{HH}=6.8 Hz, 1H, C*H* iPr),3.72 (dm, 2H, ³J_{HH}=5.27 Hz, C*H*₂-CH=CH₂), 4.99 (dm, ³J_{HH}=17.33 Hz (trans), 1H, CH₂-CH=C*H*ₐH_{b}), 5.13 (dm, ³J_{HH}=10.18 Hz (cis), 1H, CH₂-CH=CC*H*_{b}Hₐ), 6.09 (m, 1H, CH₂-C*H*=CH₂), 6.17 (s, 1H, C*H* pyrrole), 7.30 (m, 1H, p-C*H*, Ph), 7.43 (m, 4H, o-C*H* + m-C*H*, Ph).
**RMN ¹³C (CDCl₃**, **75.47 MHz)** : δ 23.21 (2C, *C*H₃ iPr), 27.01 (1C, *C*H iPr), 27.93 (3C, CH₃, tBu), 30.61 (1C, *C*H₂-CH=CH₂), 83.79 (1C, C^{IV}, tBu), 108.29 (1C, *C*H pyrrole), 115.13 (1C, CH₂-CH=*C*H₂), 125.39 (1C, C^{IV}), 126.26 (1C, p-*C*H, Ph), 127.59 (1C, C^{IV}), 128.29 et 128.58 (2C + 2C, m-*C*H + o-*C*H, Ph), 137.55 (1C, CH₂-*C*H=CH₂), 136.30 (1 C, C^{IV}), 142.30 (1 C, C^{IV}), 150.47 (1 C, C=O).

### 2-(3-hydroxy-propyl)-5-isopropyl-3-phenyl-pyrrole-1-carboxylic acid tert-butyl ester 15.

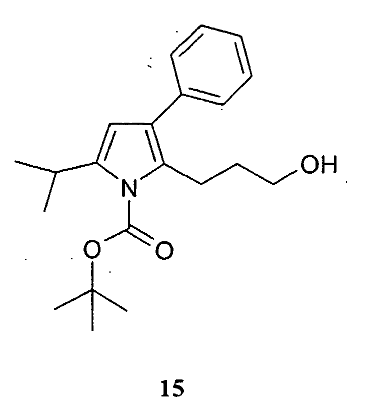

Le composé **15** est obtenu de la même manière que le composé **9**. Il est isolé avec un rendement de 68,8% après purification par chromatographie sur gel de silice (AcOEt /heptane 1 / 3 en volume - Rf0,15).
**RMN ¹H (CDCl₃, 300 MHz) :** δ 1.28 (d, ³J_{HH}=6.8 Hz, 6H, CH₃), 1.67 (s, 9H, tBu), 1.82 (m, 2H, CH₂-C*H*₂-CH₂-OH), 3.03 (t, ³J_{HH}=7.62 Hz, 2H, C*H*₂-CH₂-CH₂-OH), 3.47 (sept., ³J_{HH}=6.8 Hz, 1H, C*H* iPr), 3.53 (t, ³J_{HH}=6.22 Hz, 2H, CH₂-CH₂-C*H*₂-OH), 6.07 (s, 1H, C*H* pyrrole), 7.28 (m, 1H, p-C*H*, Ph), 7.40 (m, 4H, o-C*H* + m-C*H*, Ph).
**RMN ¹³C (CDCl₃, 75.47 MHz**) **:** δ 22.57 (1C, CH₂-*C*H₂-CH₂-OH), 23.24 (2C, *C*H₃ iPr), 27.10 (1C, *C*H iPr), 27.93 (3C, CH₃, tBu), 33.47 (1C, *C*H₂-CH₂-CH₂-OH), 62.09 (1C, CH₂-CH₂-*C*H₂-OH), 84.05 (1C, C^{IV}, tBu), 108.63 (1C, *C*H pirol), 124.93 (1C, C^{IV}), 126.31 (1C, p-*C*H, Ph), 128.43 et 128.72 (2C + 2C, m-CH + o-*C*H, Ph), 130.37 (1C, C^{IV}), 136.39 (1C, C^{IV}), 141.94 (1C, C^{IV}), 150.70 (1C, C=O).
**HRMS (IE)** : m/z calculé pour C₂₁H₂₉NO₃ ([M+.]) : théo : 343,21474 mesuré : 343,2155 (2 ppm).

### 1-(tert-Butoxycarbonyl)-2-isopropyl-4-[(2-pyrrolydin-1-ylazo)phényl]-5-(3-hydroxypropyl)pyrrole 16

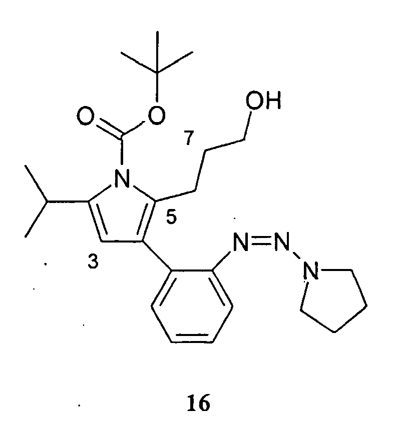

Sous atmosphère d'argon, un mélange dioxane/eau (2,5 ml, 4/1 v/v) contenant le pyrrrole **9** (89 mg; 0,26 mmol), l'acide (2-triazènyl)phénylboronique (120 mg; 0,51 mmol), du carbonate de potassium (107 mg; 0,51 mmol) et du Pd(Ph₃)₄ (30 mg; 0,1 éq) est porté à 80°C pendant une nuit. De retour à la température ambiante, le mélange est évaporé sous vide, repris à l'éther et chromatographié sur gel de silice (AcOEt / Hept 1/3) pour conduire après évaporation des fractions au produit de couplage **16** (44 mg; 0,099 mmol) sous la forme d'une huile jaune.
Rdt = 39 %
Rf = 0,17 (AcOEt/Hept 1/3)
**RMN ¹H (300 MHz, CDCl₃) :** δ * 1.20 (d, 6 H, ³J = 6.8 Hz, CH₃), 1.64 (s, 9 H, (CH₃)₃), 1.69 (m, 2 H, H₇), 1.97 (m, 4 H, Hh et Hh'), 2.32 (t large, 1H, ³J = 6.3 Hz, OH), 2.96 (t, 2 H, ³J = 6.5 Hz, H₆), 3.32 (m, 2 H, H₈), 3.46 (app quint, 1H, ³J = 6.8 Hz, CH(CH₃)), 3.50-3.90 (massif, 4H, Hg et Hg'), 5.93 (d, 1 H, ⁴J = 0.9 Hz, H₃), 7.15 (td, 1H, ³J = 7.9 Hz, ⁴J = 1.5 Hz, He ou Hd), 7.23-7.29 (m, 2H, Hf et Hd ou He), 7.40 (d, 1H, ³J = 7.9 Hz, Hc)
**RMN ¹³C (75 MHz, CDCl₃) :** δ * 23.4 (CH₃), 23.8 (Ch et Ch'), 24.1 (C6), 27.1 [*C*H(CH₃)₂], 28.0 [(CH₃)₃], 32.6 (C7), 47.6 (signal large Cg et Cg'), 61.9 (C8), 83.4 [C(CH₃)₃], 111.2 (C3), 117.5 (Cc), 123.6, 130.7, 130.8 (Ca, C4 et C5), 125.1, 127.7, 131.3 (Ce, Cd et Cf), 141.2 (C2), 149.6 (Cb ou NC=O), 150.8 (NC=O ou Cb)
**Les attributions sont confirmées par corrélation ¹H ↔ ¹³C*

| | | |
|---|---|---|
| **HRMS (FAB+)**: | m/z calculé pour C₂₅H₃₇N₄O₃ ([M+H]⁺) : | 441,2866 |
| | Mesuré: | 4.41,2863 |

### 3-(2-Amino-phenyl)-2-(3-hydroxy-propyl)-5-isopropyl-pyrrole-1-carboxylic acid tert-butyl ester 17.

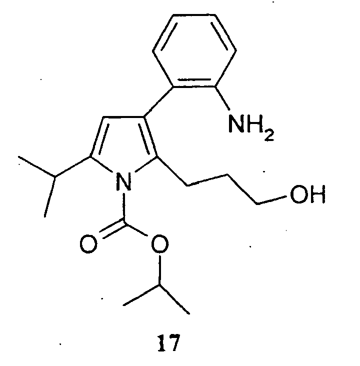

Dans un réacteur d'autoclave, le composé **16** (0.340g - 0,772 mmol) est mis en présence d'une masse équivalente de nickel de Raney (préalablement et successivement lavé avec de l'eau dégazée jusqu'à pH neutre, avec de l'éthanol absolu puis avec du méthanol) dans un mélange méthanol /acétate d'éthyle. Le mélange est porté à 25°C sous 20 bar de dihydrogène durant une nuit. La suspension est ensuite filtrée sur cellite. La cellite est rincée à l'éther. Le filtrat obtenu est ensuite évaporé. Le résidu obtenu est purifié par chromatographie sur colonne de gel de silice (AcOEt / heptane 1 / 2 en volume - Rf 0,1). Le composé **17** est isolé avec un rendement de 80,2% (0,222g) sous la forme d'une huile légèrement rouge.
**RMN ¹H (CDCl₃, 300 MHz) :** δ 1.22 (d, ³J_{HH}=5.49 Hz, 6H, C*H*₃ iPr), 1.65 (s, 9H, C*H*3 tBu), 1.68 (m, 2H, CH₂-C*H*₂-CH₂-OH), 2.83 (t, ³J_{HH}=7.11 Hz, 2H, C*H*₂-CH₂-CH₂-OH), 3.43 (t, ³J_{HH}=6.11 Hz, 2H, CH₂-CH₂-C*H*₂-OH), 3.48 (m, 1H, C*H* iPr), 5.96 (s, 1H, C*H* pyrrole), 6.73-6.80 (m, 2H, C*H* arom.), 7.05-7.13 (m, 2H, C*H* arom.).
**RMN ¹³C (CDCl₃, 75.47 MHz) :** δ 22.60 (1C, *C*H₂-CH₂-CH₂-OH), 23.22 (1C, *C*H₃ *i*Pr), 27.12 (1C, *C*H *i*Pr), 27.92 (3C, *C*H₃, tBu), 32.88 (1C, CH₂ *C*H₂-CH₂-OH), 61.47 (1C, CH₂-CH₂-*C*H₂-OH), 84.05 (1C, *C*^{IV} tBu), 108.43 (1C, *C*H arom. pyrrole), 115.23 (1C, *C*H arom.), 118.46 (1C, *C*H arom.), 121.31 (1C, *C*^{IV} arom. pyrrole), 121.80 (1C, *C*^{IV} arom. Ph), 128.29 (1C, *C*H arom.), 131.09 (1C, *C*H arom.), 131.45 (1C, CH₂-*C*^{IV} arom. pyrrole), 142.50 (1C, iPr-*C*^{IV} arom. pyrrole), 144.49 (1C, N-*C*^{IV} arom. Ph), 150.61 (1C, *C*O Boc).
**HRMS (IE):** m/z calculé pour C₂₁H₃ON₂O₃ ([M+.]) : théo : 358,22564 mesuré : 358,2218 (10 ppm).

### 3-(5-Isopropyl-3-phenyl-1H-pyrrol-2-yl)-propan-1-ol 18.

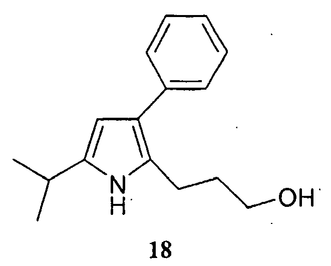

Dans un ballon de 100 mL contenant **15** (0,197g, 0,57 mmol) sont ajoutés 3,7 mL (8,7 mmol) d'une solution anhydre de méthanolate de sodium 2,33 M dans le méthanol. La solution est laissée sous agitation à la températue ambiante durant 72h. Le solvant est ensuite évaporé à l'évaporateur rotatif. Au résidu obtenu sont ajoutés 5 mL d'une solution aqueuse saturée de chlorure d'ammonium. La solution ainsi obtenue est extraite à l'éther. Après séparation des phases, la phase organique est lavée à la saumure puis séchée sur MgSO₄. Après séparation des sels de magnésium par filtration, l'éther est évaporé. Le résidu obtenu est purifié par bchromatographie sur colonne de gel de silice (AcOEt / heptane 1 / 2 en volume - Rf 0,1). Le composé **18** est isolé avec un rendement de 86,4% (0,121g) sous la forme d'une huile marron.
**RMN ¹H (CDCl₃**, **300 MHz)** : δ 1.33 (d, ³J_{HH}=6.86 Hz, 6H, C*H*₃ iPr), 1.91 (m, 2H, CH₂-C*H*₂-CH₂-OH), 2.14 (s a, 1H, O*H*), 2.91 (t, ³J_{HH}=7.3 Hz, 2H, C*H*₂-CH₂-CH₂-OH), 2.95 (sept., ³J_{HH}=6.86 Hz, 1H, C*H* iPr), 3.71 (t, ³J_{HH}=6.0 Hz, 2H, CH₂-CH₂-C*H*₂-OH), 6.06 (m, 1H, C*H* pyrrole), 7.22 (m, 1H, p-C*H*, Ph), 7.42 (m, 4H, o-C*H* + m-C*H*, Ph), 8.38 (s, 1H, N*H*).
**RMN ¹³C (CDCl₃, 75.47 MHz) :** δ 22.61 (2C, *C*H₃ iPr), 22.99 (1C, CH₂-*C*H₂-CH₂-OH), 27.01 (1C, *C*H iPr), 32.43 (1C, *C*H₂-CH₂-CH₂-OH), 62.25 (1C, CH₂-CH₂-*C*H₂-OH), 103.50 (1C, *C*H pirol), 120.67 (1C, C^{IV}), 125, 04 (1C, p-*C*H, Ph), 126.13 (1C, C^{IV}), 127.72 y 128.36 (2C + 2C, m-*C*H + o-*C*H, Ph), 137.46 (1C, C^{IV}), 137.48 (1C, C^{IV}).
**HRMS (IE)** : m/z calculé pour C₁₆H₂₁NO ([M+.]) : théo : 243,16231 mesuré : 243,1607 (6 ppm).

### 3-[3-(2-Amino-phenyl)-5-isopropyl-1H-pyrrol-2-yl]-propan-1-ol 19.

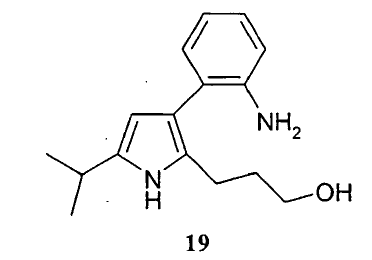

Le composé **19** est préparé à partir de **17** de la même manière que le composé **18.** Il est obtenu avec un rendement de 51,7% après purification par chromatographie sur colonne de gel de silice (AcOEt / heptane 1 / 1 en volume - Rf 0,21).
**RMN ¹H (CDCl₃, 300 MHz) :** δ 1.29 (d, ³J_{HH}=6.85 Hz, 6H, C*H*₃ iPr), 1.77 (m, 2H, CH₂-C*H*₂-CH₂-OH), 2.66 (t, ³J_{HH}=7.08 Hz, 2H, C*H*₂ -CH₂-CH₂-OH), 2.92 (hept, ³J_{HH}=6.87 Hz, 1H, C*H i*Pr), 3.59 (t, ³J_{HH}=5.93 Hz, 2H, CH₂-CH₂-C*H*₂-OH), 5.89 (m, 1H, C*H* pyrrole), 6.76-6.82 (m, 2H, C*H* arom.), 7.08-7.28 (m, 2H, C*H* arom.), 8.27 (s, 1H, N*H* pyrrole).
**RMN ¹³C (CDCl₃, 75.47 MHz) :** δ 22.36 (1C, *C*H₂-CH₂-CH₂-OH), 22.59 (2C, *C*H₃ *i*Pr), 27.04 (1C, *C*H iPr), 32.24 (1C, CH₂ *C*H₂-CH₂-OH), 61.69 (1C, CH₂CH₂-*C*H₂-OH), 108.96 (1C, *C*H arom. pyrrole), 115.19 (1C, *C*H arom.), 117.08 (1C, *C*^{IV}-C^{IV}. pyrrole), 118.44 (1C, *C*H arom. pyrrole), 123.48 (1C, *C*^{IV} arom. Ph), 127.03 (1C, CH₂-*C*^{IV} arom.), 127.40 (1C, *C*H arom.), 131.21 (1C, *C*H arom. pyrrole), 137.64 (1C, iPr-*C*^{IV} arom. pyrrole), 144.51 (1C, N*C*^{IV} arom. Ph)
**HRMS (IE) :** m/z calculé pour C₁₆H₂₂N₂O ([M+.]) : théo 258,17321 mesuré : 258,1727 (1 ppm).

### 2-(3-Hydroxy-propyl)-5-isopropyl-pyrrole-1-carboxylic acid tert-butyl ester 20

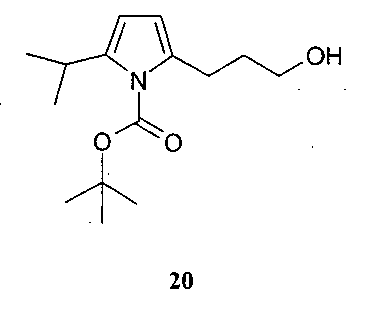

Le composé **20** est un sous produit obtenu en faible quantité lors de la synthèse de **16** à partir de **9**. Il est isolé du mélange réactionnel après l'étape d'hydrogénation permettant le passage de **16** en **17** par purification par chromatographie sur colonne de gel de silice (AcOEt / heptane 1/3 en volume - Rf 0,17).
RMN ¹H (CDCl₃, 300 MHz) : δ 1.21 (d, ³J_{HH}=6.74 Hz, 6H, C*H*₃ iPr), 1.62 (s, 9H, C*H*₃ tBu), 1.89(m, 2H + 1H, CH₂-C*H*₂-CH₂-O*H*, 2.87 (t, ³J_{HH}=7.45 Hz, 2H, C*H*₂), 3.42 (hept, ³J_{HH}=6.78 Hz, 1H, C*H i*Pr), 3.69 (t., ³J_{HH}=6.31 Hz, 2H, C*H*₂), 5.89 (m, 2H, C*H* pyrrole).
**RMN ¹³C (CDCl₃, 75.47 MHz) :** δ 23.25 (2C, *C*H₃ iPr), 25.66 (1C, CH₂-CH₂-CH₂-OH), 27.18 (1C, *C*H iPr), 27.95 (3C, *C*H₃ tBu), 32.31 (1C, *C*H₂-CH₂-CH₂-OH), 62.34 (1C, CH₂-CH₂-*C*H₂-OH), 83.70 (1C, *C*^{IV} tBu), 106.55 (1C, *C*H pyrrole), 109.20 (1C, *C*H pyrrole), 135.17 (1C, CH₂-*C*^{IV} pyrrole), 142.61 (1C, iPr-*C*^{IV} pyrrole), 150.61 (1C, *C*O Boc).

### 3-(5-Isopropyl-1H-pyrrol-2-yl)-propan-1-ol 21.

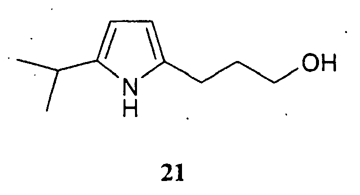

Le composé **21** est préparé à partir de **20** de la même manière que le composé **18**. Le composé **21** a été isolé avec un rendement de 97,8% après purification par chromatographie sur colonne de gel de silice (AcOEt / heptane 1 / 1 en volume - Rf 0,37).
**RMN ¹H (CDCl₃, 300 MHz) :** δ 1.29 (d, ³J_{HH}=6.86 Hz, 6H, C*H*₃ iPr), 1.90(m, 2H, CH₂-C*H*₂ -CH₂-OH), 2.07 (s, 1H, O*H*), 2.71 (t, ³J_{HH}=7.35 Hz, 2H, C*H*₂-CH₂-CH₂-OH), 2.91 (hept, ³J_{HH}=6.87 Hz, 1H, C*H* iPr), 3.73 (t., ³J_{HH}=6.12 Hz, 2H, CH₂CH₂-C*H*₂-OH), 5.83 (m, 2H, C*H* pyrrole), 8.10 (s, 1 H, N*H).*
**RMN ¹³C (CDCl₃, 75.47 MHz) :** δ 23.69 (2C, *C*H₃ iPr), 24.29 (1C, CH₂-*C*H₂-CH₂-OH), 27.06 (1C, *C*H iPr), 32.34 (1C, *C*H₂-CH₂-CH₂-OH), 62.40 (1C, CH₂-CH₂-*C*H₂-OH), 102.60 (1C, *C*H pyrrole), 104.54 (1C, *C*H pyrrole), 130.21 (1C, CH₂-*C*^{IV} pyrrole), 137.86 (1C, *i*Pr-*C*^{IV} pyrrole).
**HRMS (IE)** : m/z calculé pour C₁₀H₁₇NO ([M+]) : théo 167,13101 mesuré : 167,1320 (5 ppm).

### II. Analyses biologiques

**But** : Etant donné le rôle de la tubuline dans la formation du fuseau mitotique, nous avons analysé par différentes méthodes, l'effet des composés selon la présente invention, sur le cycle cellulaire de cellules murines : la lignée tumorale murine de mélanome, la lignée B16 F1 et la lignée mel a (mélanocytes) et la lignée L1210 de leucémie de souris.

### 1- Effet des composés 17, 18,19 et 21 sur la croissance cellulaire

Nous avons dans un premier temps évalué l'effet des composés 17, 18, 19 sur la croissance des trois lignées cellulaires par un test "MTT". Le principe du test MTT consiste à mesurer l'activité de la succinate déshydrogénase mitochondriale des cellules vivantes. Cette enzyme transforme le MTT (ou Bromure de 3(4.5-diméthylthiazol-2-yl)-2.5-diphényltétrazolium), de couleur jaune, en cristaux de formazan violets et insolubles. Après dissolution des cristaux dans du diméthylsulfoxyde (DMSO), l'absorbance est mesurée à 540 nm par spectrophotométrie. Les densités optiques obtenues sont directement proportionnelles au nombre de cellules vivantes. L'effet cytotoxique d'un échantillon est évalué par le pourcentage de cellules vivantes en présence de cet échantillon, par rapport aux cellules traitées avec le solvant seul. Cette méthode permet de mesurer la proportion de cellules vivantes dans une population cellulaire donnée. Par comparaison avec des cellules non traitées, nous avons ainsi déterminé la concentration de chaque composé qui induit une inhibition de la croissance cellulaire de 50%. Les résultats obtenus sont consignés dans le tableau 1. Les CI₅₀ vont de 50 à 100µM.

| **Tableau 1**. Comparaison de l'effet des composés sur la croissance des cellules B16 F1, des mélanocytes mel a et leucémies de souris L1210. | | | | |
|---|---|---|---|---|
| **Composés** | | CI₅₀* (µM) ± sd | | |
| | | B16 F1 | Mel a | L1210 |
| **17** | 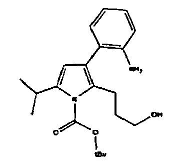 | 88,7 ± 7,6 | 69,7 ± 0,9 | 53,7 ± 0,7 |
| **19** | 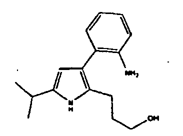 | 106,7 ± 1,8 | 116,1 ± 3,2 | 17,7 ± 4,5 |
| **18** | 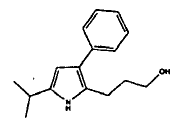 | 171,0 ± 10,8 | n.d. | 33,2 ± 1,3 |
| **21** | 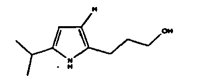 | >400 | n.d. | >400 |

| | | | | |
|---|---|---|---|---|
| La CI₅₀ est la concentration qui inhibe 50% de la croissance cellulaire. Les valeurs données représentent la moyenne ± écart-type de 3 expériences différentes réalisées en triplicat. | | | | |

Le caractère antiprolifératif des composés varie en fonction de la lignée cellulaire étudiée et du composé analysé On observe une meilleure IC50 en présence des composés 19 sur les cellules L1210 (leucémie de souris).
Le composé **21** n'est pas fonctionnalisé en position 3 du noyau pyrrole. Il sert de référence et démontre que la position 3 du noyau pyrrole est une position stratégique pour obtenir une activité biologique.

### 2- Effet des composés sur le déroulement du cycle cellulaire

Afin d'approcher le mécanisme d'action de ces composés, nous avons ensuite étudié leur effet sur le déroulement du cycle cellulaire chez les cellules mélanomes de souris (B16) et leucémie de souris (L1210). Pour cela, nous avons comparé la distribution des cellules traitées ou non dans chaque phase du cycle. Les cellules sont traitées pendant 24 heures avec des concentrations se situant autour de la CI₅₀ de chaque composé. Elles sont alors fixées et perméabilisées avec de l'éthanol. Après incubation avec de l'iodure de propidium qui marque l'ADN cellulaire, le contenu en ADN des cellules est ensuite analysé par cytométrie en flux.
Les résultats obtenus sont rassemblés dans le tableau 2.

| **Tableau 2**. Effet des composés sur le déroulement du cycle cellulaire. Analyse par cytométrie en flux après marquage à l'iodure de propidium. | | | | | | |
|---|---|---|---|---|---|---|
| | % de cellules B16^{a} | | | % de cellules L1210 | | |
| | En phase G1 | En phase S | En phase G2/M | En phase G1 | En phase S | En phase G2/M |
| Contrôle | 47,8 ± 6,2 | 43,7 ± 2,9 | 8,5 ± 4,3 | 29,4 | 60,7 | 10,0 |

| Composé **17** | | | | | | |
|---|---|---|---|---|---|---|
| 40µM | - | - | - | 54,9 | 21,9 | 23,2 |
| 50µM | 58,0 | 16,6 | 25,4 | 39,2 | 17,2 | 43,6 |
| 70µM | 40,8 | 37 | 22,3 | - | - | - |
| 100µM | 43,3 | 40,3 | 16,5 | - | - | - |

| Composé **19** | | | | | | |
|---|---|---|---|---|---|---|
| 50µM | 78,7 | 14,1 | 5,2 | 49,5 | 26,5 | 24 |
| 75µM | 56,1 | 2,8 | 31,9 | 39,8 | 21,4 | 38,8 |

| Composé **18** | | | | | | |
|---|---|---|---|---|---|---|
| 40µM | - | - | - | 32,7 | 54,7 | 12,6 |
| 55µM | - | - | - | 30,9 | 47,2 | 21,9 |
| 75µM | 65,0 | 11,3 | 12,1 | - | - | - |
| 125µM | 50,0 | 5,2 | 32,0 | - | - | - |
| 150µM | 42,1 | 21,8 | 36,0 | - | - | - |
| ^{a} Pour les composé 19 et 18, sauf à 150µM, les valeurs indiquées représentent la moyenne ± sd de 2 expériences au moins. Pour le composé 17, la valeur indiquée est celle d'une seule expérience. | | | | | | |

Pour des concentrations situées au-dessous de la CI₅₀, nous observons une accumulation des cellules en phase G1 (gap 1), ainsi qu'une diminution de la quantité de cellules en phase S (synthèse d'ADN). Les cellules traitées avec des concentrations de l'ordre de la CI₅₀, ou avec des concentrations supérieures, présentent quant à elles une plus forte proportion de cellules en phase G2/M (gap 2/mitose) (de l'ordre de 3 fois plus pour le composé 2). La proportion de cellules en phase G1 semble par contre être « normalisée » c'est-à-dire que le taux de cellules en G1 n'est pas modifié. Pour les concentrations les plus élevées, la quantité de cellules en phase S paraît également se rapprocher des valeurs des cellules contrôle.

La méthode de marquage à l'iodure de propidium ne permet pas de distinguer les cellules qui répliquent leur ADN activement. Nous ne pouvons donc pas affirmer que les cellules observées en phase S dans les cellules traitées (c'est-à-dire ayant une quantité d'ADN de 4n) sont bien des cellules qui répliquent activement leur ADN ou si elles sont simplement bloquées en phase S.

Pour tenter d'élucider ce problème, nous avons incubé des cellules traitées ou non avec du Bromo desoxy Uridine pendant une courte durée (pulse). Le BrdU s'incorpore dans l'ADN en réplication des cellules. Après fixation, les cellules sont perméabilisées puis incubées en présence d'un anticorps anti-BrdU marqué avec un fluorochrome. Les cellules sont également marquées avec de l'iodure de propidium, puis analysées par cytométrie en flux.

Les profils obtenus (tableau 2) montrent que les cellules traitées avec des concentrations égales ou supérieures à la CI₅₀ n'incorporent pas du tout de BrdU. Ces cellules ne répliquent donc plus leur ADN.

## Revendications

1. Composé représenté par la formule générale I suivante 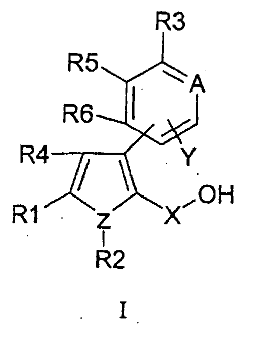 dans laquelle :
Z représente N, O ou S
R1, R3, R4, R5 et R6 représentent indépendamment l'un de l'autre un atome d'hydrogène, un atome de fluor, un groupe alkyle en C₁-C₁₂ linéaire ou ramifié, cycloalkyle en C₃-C₁₂, alcényle en C₂-C₁₂ linéaire ou ramifié, CH₂-B ou CH₂-CH₂-B dans lesquels B représente
- un groupe phényle substitué ou non par un ou plusieurs groupes choisis parmi un groupe alkyle en C₁-C₆ linéaire ou ramifié, alkoxy en C₁-C₆linéaire ou ramifié, NH₂, NO₂, CN, COOH, CO₂(alkyle) en C₁-C₆ linéaire ou ramifié, CONH₂, CONH(alkyle) en C₁-C₆ linéaire ou ramifié, CON(alkyle)₂ en C₁-C₆ linéaire ou ramifié Cl, Br, I, OH, COCF₃, OSO₂CF₃;
- naphtyle;
- anthracényle;
- 9H-fluorényle substitué ou non en position 9 par un ou deux groupe alkyle en C₁-C₁₂ linéaire ou ramifié;
- anisyle ou pyridinyle,
R2 représente H, un groupe alkyle en C₁-C₁₂ linéaire ou ramifié, phényle, benzyle, CO₂alkyle en C₁-C₆ linéaire ou ramifié, CO₂benzyle, CO₂alcény en C₂-C₆ linéaire ou ramifié, tosyle, mésyle, 9-fluorenylmethoxycarbonyl Fmoc, NH₂ ou NH(alkyle) en C₁-C₆ linéaire ou ramifié, N(alkyle)₂ en C₁-C₆ linéaire ou ramifié ou NH tertiobutyloxycarbonyl ou NHCO₂CH₂ phényle ou R2 est absent quand Z représente O.
X représente un groupe alkyle en C₁-C₁₂ linéaire ou ramifié, hydroxyalkyle en C₁-C₁₂ linéaire ou ramifié, ou aminoalkyle en C₁-C₁₂ linéaire ou ramifié.
A représente un groupe CH, un atome d'azote ou un groupe NL⁺ dans lequel L représente un groupe alkyle en C₁-C₁₂ linéaire ou ramifié.
Y représente un atome d'hydrogène, un groupe alkyle en C₁-C₁₂, linéaire ou ramifié, cycloalkyle en C₃-C₁₂, OH, CN, N₃, alcoxy en C₁-C₁₂ linéaire ou ramifié, hydroxyalkyle en C₁-C₁₂ linéaire ou ramifié, aminoalkyle en C₁-C₁₂ linéaire ou ramifié, N₂⁺, NZ1-NHZ2, NH-NZ1Z2 ou NZ1Z2 dans lesquels
Z1 et Z2 représentent indépendamment l'un de l'autre un atome d'hydrogène, un groupe alkyle en C₁-C₁₂ linéaire ou ramifié ; cycloalkyle en C₃-C₁₂ ; phényle substitué ou non par un ou plusieurs groupes choisis parmi un groupe alkyle en C₁-C₆ linéaire ou ramifié, alkoxy en C₁-C₆ linéaire ou ramifié, NH₂, CN, COOH, CO₂(alkyle) en C₁-C₆ linéaire ou ramifié, CONH₂, CONH(alkyle) en C₁-C₆ linéaire ou ramifié, CON(alkyle)₂ en C₁-C₆ linéaire ou ramifié CI, Br, I, OH, COCF₃ ou OSO₂CF₃ ; benzyle ; anisyle ; pyridinyle ; C(O)-W ; C(S)-W ou C(NH)-W, dans lesquels
W représente un groupe alkyle en C₁-C₁₂ linéaire ou ramifié, cycloalkyle en C₃-C₁₂, alcoxy en C₁-C₁₂ linéaire ou ramifié, alkylthio en C₁-C₁₂ linéaire ou ramifié ou NQQ1,
dans lesquels
Q et Q1 représentent indépendamment l'un de l'autre un atome d'hydrogène, un groupe alkyle en C₁-C₁₂ linéaire ou ramifié, cycloalkyle en C₃-C₁₂, ou CH(M)CO₂M1 dans lesquels
M et M1 représentent indépendamment l'un de l'autre un atome d'hydrogène, un groupe alkyle en C₁-C₁₂ linéaire ou ramifié, cycloalkyle en C₃-C₁₂, alcényle en C₂-C₁₂ linéraire ou ramifié, phényle, benzyle, CH₂-B ou CH₂-CH₂-B dans lesquels B est tel que défini ci-dessus,
à l'exception des composés de formules suivantes : 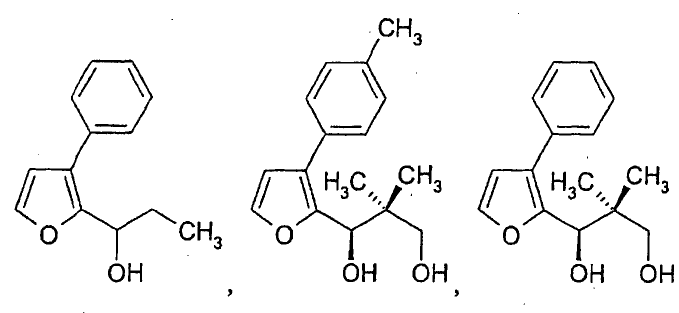 et

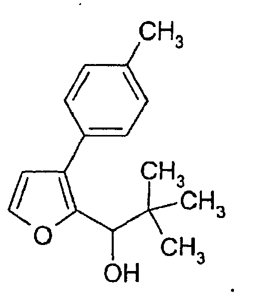

2. Composé selon la revendication 1 dans lequel Z représente N.

3. Composé selon l'une quelconque des revendications précédentes dans lequel A représente le groupe CH.

4. Composé selon l'une quelconque des revendications précédentes dans lequel Y représente un groupe NH₂ ou un atome d'hydrogène.

5. Composé selon l'une quelconque des revendications précédentes dans lequel X représente un groupe alkyle en C₁-C₁₂ linéaire ou ramifié, avantageusement, un groupe propyle.

6. Composé selon l'une des revendications précédentes **caractérisé en ce qu'**il est choisi dans le groupe constitué par 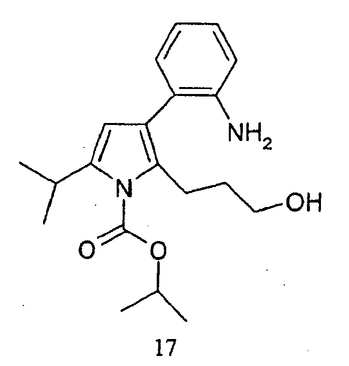 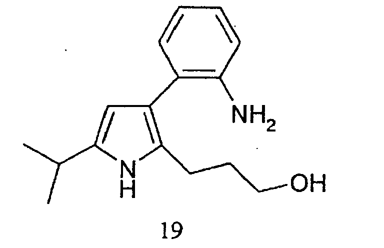 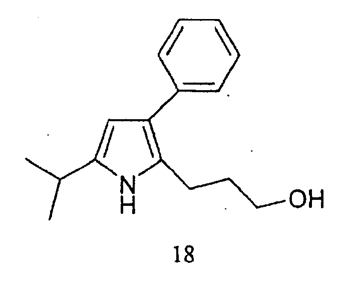 et

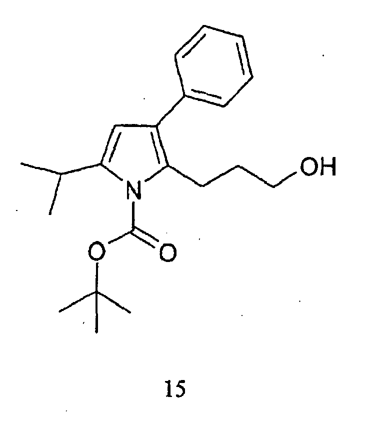

7. Procédé de préparation des composés selon l'une quelconque des revendications précédentes comprenant les étapes de :
a)synthèse d'un motif pyrrolylalkylcarbinole, furanylalkylcarbinole ou thiophénylalkylcarbinole de formule générale II suivante 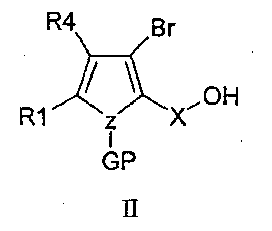 dans laquelle Z, X, R₁ et R4 sont tels que définis dans la revendication 1 et GP représente un groupe protecteur de l'azote dans le cas ou Z représente N, ou est absent lorsque Z représente O ou S,
b) fonctionnalisation du motif pyrrolylalkylcarbynol furanylalkylcarbinole ou thiophénylalkylcarbinole en introduisant un motif arylique ou hétéroarylique en position 3 du cycle pyrole, furane ou thiophéne.

8. Procédé selon la revendication 7 dans le cas où X représente (CH₂)₃ **caractérisé en ce que** l'étape a) consiste en la cyclodéshydratation d'aminocétones β-γ insaturées de formule III suivante : 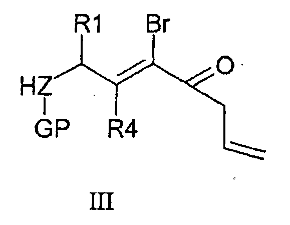 dans laquelle Z, R1, R4 et GP sont tels que définis dans la revendication 7 afin d'obtenir le produit de formule IV suivante 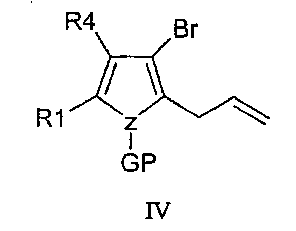 dans laquelle Z, R1, R4 et GP sont tels que définis dans la revendication 7,
suivi de l'introduction de la fonction alcool par hydroboration/oxydation du produit de formule IV afin d'obtenir le produit de formule II tel que défini dans la revendication 7.

9. Procédé selon l'une quelconque des revendications 7 ou 8 dans le cas où R2, R3, R4, R5 et R6 représentent un atome d'hydrogène, Y représente un groupe NH₂, A représente un groupe CH, X représente (CH₂)₃ et Z représente N, **caractérisé en ce que** l'étape b) consiste en :
- un couplage mixte pallado-catalysée de type Suzuki-Miyaura du composé de formule II dans lequel R4 représente un atome d'hydrogène, X représente (CH₂)₃, Z représente N et GP représente un groupe protecteur de l'azote et l'acide 2-triazène boronique de façon à obtenir un composé de formule V suivante : 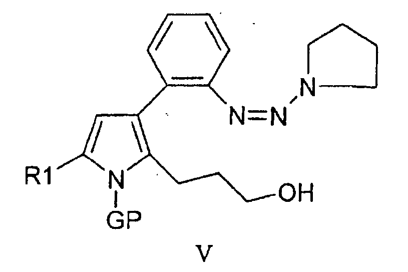
dans laquelle GP représente un groupe protecteur de l'azote et R1 est tel que défini dans la revendication 1,
- une déprotection de la fonction triazène afin d'obtenir le groupe de formule VI suivant 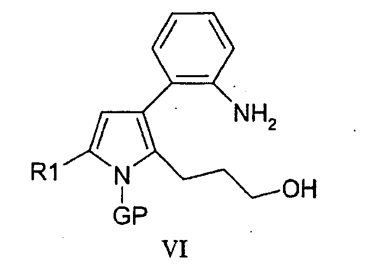
dans laquelle GP représente un groupe protecteur de l'azote et R1 est tel que défini dans la revendication 1
- une déprotection spécifique du groupement protecteur GP afin d'obtenir le composé de formule I dans laquelle R2, R3, R4, R5 et R6 représentent un atome d'hydrogène, Y représente un groupe NH₂, A représente un groupe CH, X représente (CH₂)₃, Z représente N et R1 est tel que défini dans la revendication 1.

10. Composition pharmaceutique comprenant un composé selon l'une quelconque des revendications 1 à 6 ou un composé choisi parmi 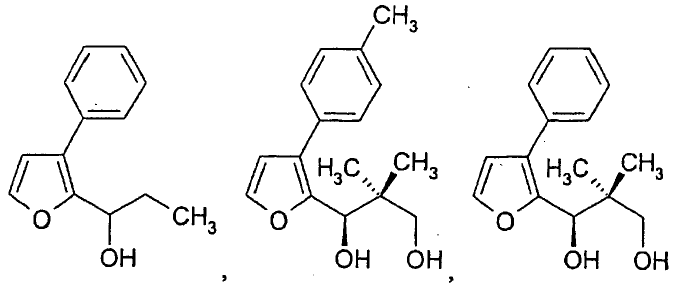 et 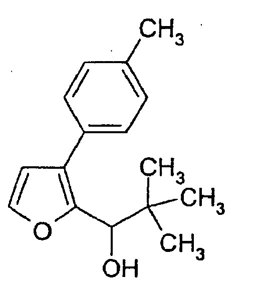 et un excipient pharmaceutiquement acceptable.

11. Composé selon l'une quelconque des revendications 1 à 6 ou choisi parmi 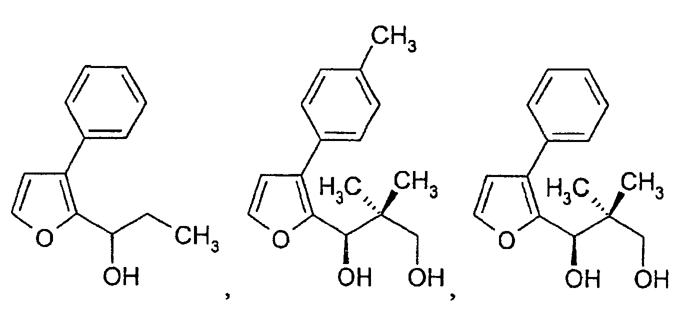 et 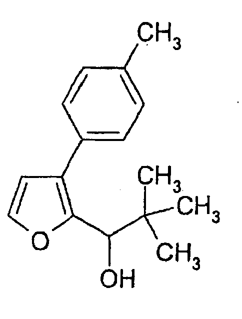 pour son utilisation en tant que médicament.

12. Composé selon la revendication 11 pour son utilisation en tant que médicament antimitotique, avantageusement antitumoral.

13. Composé selon la revendication 12 pour son utilisation en tant que médicament destiné au traitement ou à la prévention du cancer.

14. Composé de formule générale V 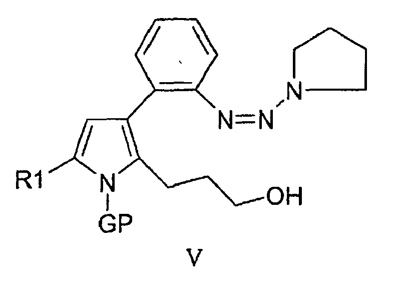 dans lequel GP représente un groupe protecteur de l'azote et R1 représente un atome d'hydrogène, un atome de fluor, un groupe alkyle en C₁-C₁₂ linéaire ou ramifié, cycloalkyle en C₃-C₁₂, alcényle en C₂-C₁₂ linéaire ou ramifié, CH₂-B ou CH₂-CH₂-B
dans lesquels B représente un groupe phényle substitué ou non par un ou plusieurs groupes choisis parmi un groupe alkyle en C₁-C₆ linéaire ou ramifié, alkoxy en C₁-C₆ linéaire ou ramifié, NH₂, NO₂, CN, COOH, CO₂(alkyle) en C₁-C₆ linéaire ou ramifié, CONH₂, CONH(alkyle) en C₁-C₆ linéaire ou ramifié, CON(alkyle)₂ en C₁-C₆ linéaire ou ramifié Cl, Br, I, OH, COCF₃, OSO₂CF₃ ; naphtyle; anthracényle ; 9H-fluorényle substitué ou non en position 9 par un ou deux groupe alkyle en C₁-C₁₂ linéaire ou ramifié ; anisyle ou pyridinyle.

15. Composé de formule générale VI 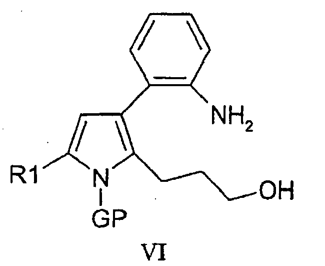 dans lequel GP représente un groupe protecteur de l'azote et R1 représente un atome d'hydrogène, un atome de fluor, un groupe alkyle en C₁-C₁₂ linéaire ou ramifié, cycloalkyle en C₃-C₁₂, alcényle en C₂-C₁₂ linéaire ou ramifié, CH₂-B ou CH₂-CH₂-B
dans lesquels B représente un groupe phényle substitué ou non par un ou plusieurs groupes choisis parmi un groupe alkyle en C₁-C₆ linéaire ou ramifié, alkoxy en C₁-C₆ linéaire ou ramifié, NH₂, NO₂, CN, COOH, CO₂(alkyle) en C₁-C₆ linéaire ou ramifié, CONH₂, CONH(alkyle) en C₁-C₆ linéaire ou ramifié, CON(alkyle)₂ en C₁-C₆ linéaire ou ramifié CI, Br, I, OH, COCF₃, OSO₂CF₃ ; naphtyle; anthracényle; 9H-fluorényle substitué ou non en position 9 par un ou deux groupe alkyle en C₁-C₁₂ linéaire ou ramifié ; anisyle ou pyridinyle.

## Claims

1. Compound represented by the general formula I below 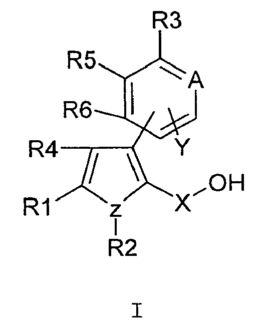 in which:
Z represents N, O or S,
R1, R3, R4, R5 and R6 represent, independently of each other, a hydrogen atom, a fluorine atom, a linear or branched C₁-C₁₂ alkyl group, C₃-C₁₂ cycloalkyl group, linear or branched C₂-C₁₂ alkenyl group, CH₂-B or CH₂-CH₂-B in which B represents
- a phenyl group optionally substituted with one or more groups chosen from a linear or branched C₁-C₆ alkyl group, linear or branched C₁-C₆ alkoxy group, NH₂, NO₂, CN, COOH, linear or branched C₁-C₆ CO₂ (alkyl) group, CONH₂, linear or branched C₁-C₆ CONH(alkyl) group, linear or branched C₁-C₆ CON(alkyl)₂ group, Cl, Br, I, OH, COCF₃ and OSO₂CF₃;
- naphthyl;
- anthracenyl;
- 9H-fluorenyl optionally substituted in position 9 with one or two linear or branched C₁-C₁₂ alkyl groups;
- anisyl or pyridyl,
R2 represents H, a linear or branched C₁-C₁₂ alkyl group, phenyl, benzyl, linear or branched C₁-C₆ CO₂alkyl group, CO₂benzyl, linear or branched C₂-C₆ CO₂alkenyl group, tosyl, mesyl, 9-fluorenylmethoxycarbonyl (Fmoc), NH₂ or linear or branched C₁-C₆ NH(alkyl) group, linear or branched C₁-C₆ N(alkyl)₂ group or NH-tert-butyloxycarbonyl or NHCO₂CH₂phenyl or R2 is absent when Z represents O,
X represents a linear or branched C₁-C₁₂ alkyl group, linear or branched C₁-C₁₂ hydroxyalkyl group or linear or branched C₁-C₁₂ aminoalkyl group,
A represents a CH group, a nitrogen atom or a group NL⁺ in which L represents a linear or branched C₁-C₁₂ alkyl group,
Y represents a hydrogen atom, a linear or branched C₁-C₁₂ alkyl group, C₃-C₁₂ cycloalkyl group, OH, CN, N₃, linear or branched C₁-C₁₂ alkoxy group, linear or branched C₁-C₁₂ hydroxyalkyl group, linear or branched C₁-C₁₂ aminoalkyl group, N₂⁺, NZ1-NHZ2,NH-NZ1Z2 or NZ1Z2 in which
Z1 and Z2 represent, independently of each other, a hydrogen atom, a linear or branched C₁-C₁₂ alkyl group; C₃-C₁₂ cycloalkyl group; phenyl optionally substituted with one or more groups chosen from a linear or branched C₁-C₆ alkyl group, linear or branched C₁-C₆ alkoxy group, NH₂, CN, COOH, linear or branched C₁-C₆ CO₂ (alkyl) group, CONH₂, linear or branched C₁-C₆ CONH(alkyl) group, linear or branched C₁-C₆ CON (alkyl)₂ group, Cl, Br, I, OH, COCF₃ or OSO₂CF₃; benzyl; anisyl; pyridyl; C(O)-W; C(S)-W or C(NH)-W, in which
W represents a linear or branched C₁-C₁₂ alkyl group, C₃-C₁₂ cycloalkyl group, linear or branched C₁-C₁₂ alkoxy group, linear or branched C₁-C₁₂ alkylthio group or NQQ1, in which
Q and Q1 represent, independently of each other, a hydrogen atom, a linear or branched C₁-C₁₂ alkyl group, C₃-C₁₂ cycloalkyl group or CH(M)CO₂M1 in which
M and M1 represent, independently of each other, a hydrogen atom, a linear or branched C₁-C₁₂ alkyl group, C₃-C₁₂ cycloalkyl group, linear or branched C₂-C₁₂ alkenyl group, phenyl, benzyl, CH₂-B or CH₂-CH₂-B in which B is as defined above,
with the exception of the compounds of the following formulae: 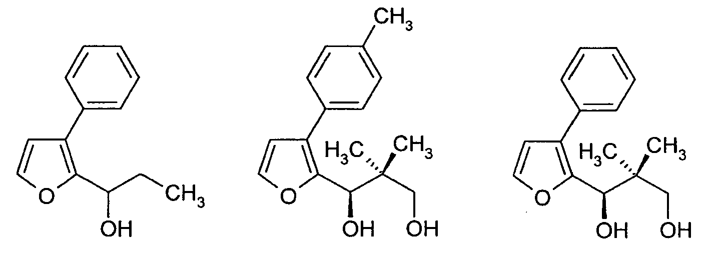 and

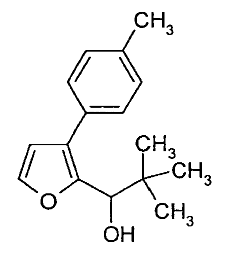

2. Compound according to Claim 1, in which Z represents N.

3. Compound according to either of the preceding claims, in which A represents a CH group.

4. Compound according to any one of the preceding claims, in which Y represents an NH₂ group or a hydrogen atom.

5. Compound according to any one of the preceding claims, in which X represents a linear or branched C₁-C₁₂ alkyl group, advantageously a propyl group.

6. Compound according to one of the preceding claims, **characterized in that** it is chosen from the group constituted by 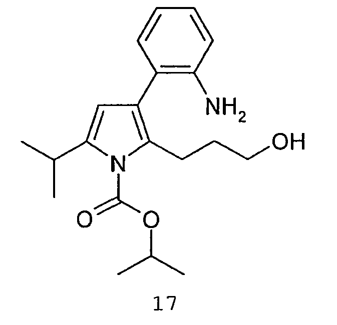 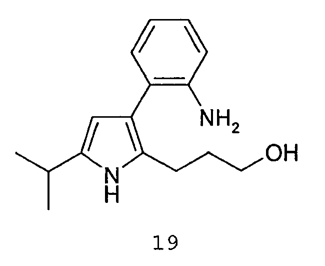 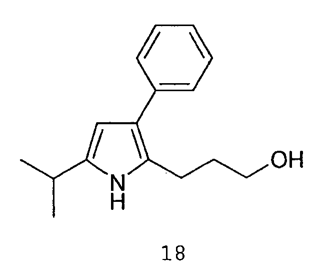 and

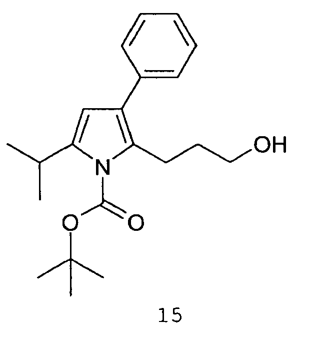

7. Process for preparing compounds according to any one of the preceding claims, comprising the steps of:
a) synthesis of a pyrrolylalkylcarbinol, furylalkylcarbinol or thiophenylalkylcarbinol unit of general formula II below 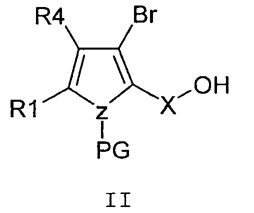 in which Z, X, R1 and R4 are as defined in Claim 1 and PG represents a nitrogen-protecting group when Z represents N, or is absent when Z represents O or S,
b) functionalization of the pyrrolylalkylcarbinol, furylalkylcarbinol or thiophenylalkylcarbinol unit by introducing an aryl or heteroaryl unit into position 3 of the pyrrole, furan or thiophene ring.

8. Process according to Claim 7, in the case where X represents (CH₂)₃, **characterized in that** step a) consists of the cyclodehydration of β,γ-unsaturated amino ketones of formula III below: 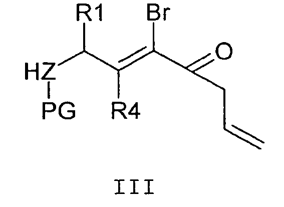 in which Z, R1, R4 and PG are as defined in Claim 7, in order to obtain the product of formula IV below 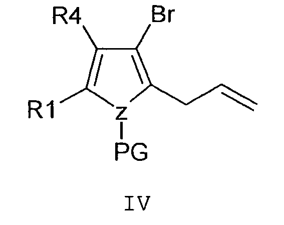 in which Z, R1, R4 and PG are as defined in Claim 7, followed by the introduction of the alcohol function by hydroboration/oxidation of the product of formula IV in order to obtain the product of formula II as defined in Claim 7.

9. Process according to either of Claims 7 and 8, in the case where R2, R3, R4, R5 and R6 represent a hydrogen atom, Y represents an NH₂ group, A represents a CH group, X represents (CH₂)₃ and Z represents N, **characterized in that** step b) consists of:
- a palladium-catalysed mixed coupling of Suzuki-Miyaura type, of the compound of formula II in which R4 represents a hydrogen atom, X represents (CH₂)₃, Z represents N and PG represents a nitrogen-protecting group and of 2-triazineboronic acid so as to obtain a compound of formula V below: 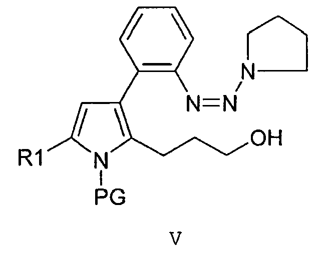
in which PG represents a nitrogen-protecting group and R1 is as defined in Claim 1,
- a deprotection of the triazine function in order to obtain the group of formula VI below 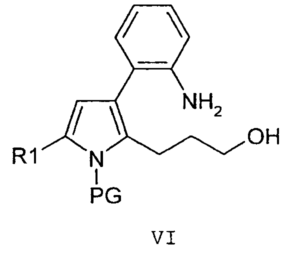
in which PG represents a nitrogen-protecting group and R1 is as defined in Claim 1,
- a specific deprotection of the protecting group PG in order to obtain the compound of formula I in which R2, R3, R4, R5 and R6 represent a hydrogen atom, Y represents an NH₂ group, A represents a CH group, X represents (CH₂)₃, Z represents N and R1 is as defined in Claim 1.

10. Pharmaceutical composition comprising a compound according to any one of Claims 1 to 6 or a compound chosen from 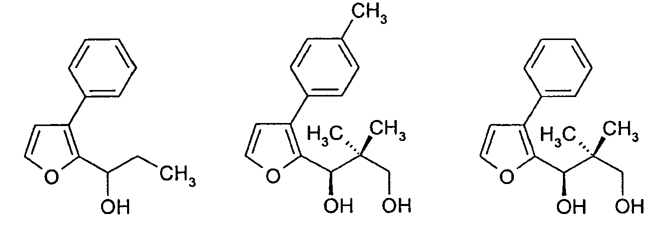 and 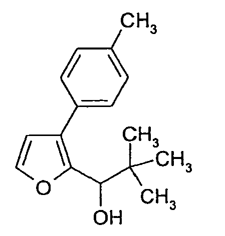 and a pharmaceutically acceptable excipient.

11. Compound according to any one of Claims 1 to 6 or chosen from 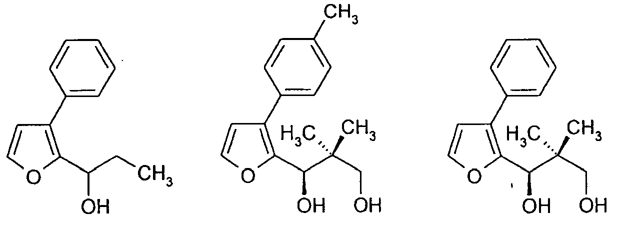 and 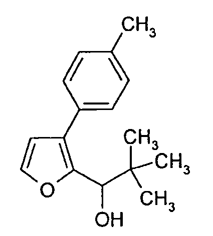 for its use as a medicament.

12. Compound according to Claim 11, for its use as an antimitotic and advantageously antitumoral medicament.

13. Compound according to Claim 12, for its use as a medicament for treating or preventing cancer.

14. Compound of general formula V 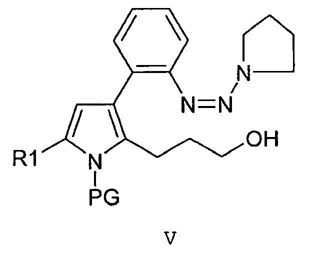 in which PG represents a nitrogen-protecting group and R1 represents a hydrogen atom, a fluorine atom, a linear or branched C₁-C₁₂ alkyl group, C₃-C₁₂ cycloalkyl group, linear or branched C₂-C₁₂ alkenyl group, CH₂-B or CH₂-CH₂-B in which B represents a phenyl group optionally substituted with one or more groups chosen from a linear or branched C₁-C₆ alkyl group, linear or branched C₁-C₆ alkoxy group, NH₂, NO₂, CN, COOH, linear or branched C₁-C₆ CO₂(alkyl) group, CONH₂, linear or branched C₁-C₆ CONH(alkyl) group, linear or branched C₁-C₆ CON(alkyl)₂ group, Cl, Br, I, OH, COCF₃, OSO₂CF₃; naphthyl; anthracenyl; 9H-fluorenyl optionally substituted in position 9 with one or two linear or branched C₁-C₁₂ alkyl groups; anisyl or pyridyl.

15. Compound of general formula VI 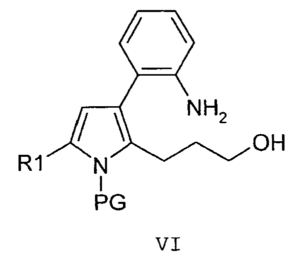 in which PG represents a nitrogen-protecting group and R1 represents a hydrogen atom, a fluorine atom, a linear or branched C₁-C₁₂ alkyl group, C₃-C₁₂ cycloalkyl group, linear or branched C₂-C₁₂ alkenyl group, CH₂-B or CH₂-CH₂-B in which B represents a phenyl group optionally substituted with one or more groups chosen from a linear or branched C₁-C₆ alkyl group, linear or branched C₁-C₆ alkoxy group, NH₂, NO₂, CN, COOH, linear or branched C₁-C₆ CO₂(alkyl) group, CONH₂, linear or branched C₁-C₆ CONH(alkyl) group, linear or branched C₁-C₆ CON (alkyl) ₂ group, Cl, Br, I, OH, COCF₃, OSO₂CF₃; naphthyl; anthracenyl; 9H-fluorenyl optionally substituted in position 9 with one or two linear or branched C₁-C₁₂ alkyl groups; anisyl or pyridyl.

## Patentansprüche

1. Verbindung, die durch die folgende allgemeine Formel I dargestellt wird 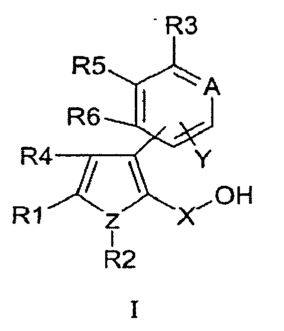 in der:
Z für N, O oder S steht,
R1, R3, R4, R5 und R6 unabhängig voneinander ein Wasserstoffatom, ein Fluoratom, eine lineare oder verzweigte (C₁-C₁₂)-Alkyl-, (C₃-C₁₂)-Cycloalkyl-, lineare oder verzweigte (C₂-C₁₂)-Alkenyl-Gruppe, eine Gruppe CH₂-B oder CH₂-CH₂-B darstellen, worin B darstellt:
- eine Phenylgruppe, die unsubstituiert oder durch eine oder mehrere Gruppen substituiert ist, die ausgewählt sind aus einer linearen oder verzweigten (C₁-C₆)-Alkyl-, linearen oder verzweigten (C₁-C₆)-Alkoxy-, NH₂-, NO₂-, CN-, COOH-, linearen oder verzweigten CO₂-((C₁-C₆)-Alkyl)-, CONH₂-, linearen oder verzweigten CONH((C₁-C₆)-Alkyl)-, linearen oder verzweigten CON((C₁-C₆)-Alkyl)₂-, Cl-, Br-, 1-, OH-, COCF₃-, OSO₂CF₃-Grupp
- Naphthyl;
- Anthracenyl;
- 9H-Fluorenyl, das unsubstituiert oder in Position 9 durch eine oder zwei lineare oder verzweigte (C₁-C₁₂)-Alkylgruppen substituiert ist;
- Anisyl oder Pyridinyl,
R₂ für H, eine lineare oder verzweigte (C₁-C₁₂)-Alkyl-, Phenyl-, Benzyl-, lineare oder verzweigte CO₂-(C₁-C₆)-Alkyl-, CO₂-Benzyl, lineare oder verzweigte CO₂-(C₂-C₆)-Alkenyl-, Tosyl-, Mesyl-, 9-Fluorenylmethoxycarbonyl(Fmoc)-, NH₂- oder lineare oder verzweigte NH((C₁-C₆)-Alkyl)-, lineare oder verzweigte N((C₁-C₆)-Alkyl)₂- oder NH-Tertiärbutyloxycarbonyl- oder NHCO₂CH₂-Phenylgruppe steht oder R2 abwesend ist, wenn Z für O steht,
X eine lineare oder verzweigte (C₁-C₁₂)-Alkyl-, lineare oder verzweigte (C₁-C₁₂)-Hydroxyalkyl- oder lineare oder verzweigte (C₁-C₁₂)-Aminoalkylgruppe darstellt,
A eine CH-Gruppe, ein Stickstoffatom oder eine Gruppe NL⁺ darstellt, in der L eine lineare oder verzweigte (C₁-C₁₂)-Alkylgruppe darstellt,
Y ein Wasserstoffatom, eine lineare oder verzweigte (C₁-C₁₂)-Alkyl-, (C₃-C₁₂)-Cycloalkyl-, OH-, CN-, N₃-, lineare oder verzweigte (C₁-C₁₂)-Alkoxy-, lineare oder verzweigte (C₁-C₁₂)-Hydroxyalkyl-, lineare oder verzweigte (C₁-C₁₂)-Aminoalkylgruppe, eine Gruppe N₂⁺, NZ1-NHZ2-, NH-NZ1Z2 oder NZ1 Z2 darstellt, worin
Z1 und Z2 unabhängig voneinander ein Wasserstoffatom, eine lineare oder verzweigte (C₁-C₁₂)-Alkyl-, (C₃-C₁₂)-Cycloalkyl-, Phenylgruppe, die unsubstituiert oder durch eine oder mehrere Gruppen substituiert ist, die ausgewählt sind aus einer linearen oder verzweigten (C₁-C₆)-Alkyl-, linearen oder verzweigten (C₁-C₆)-Alkoxy-, NH₂-, CN-, COOH-, linearen oder verzweigten CO₂((C₁-C₆)-Alkyl)-, CONH₂-, linearen oder verzweigten CONH((Cᵢ-C₆)-Alkyl)-, linearen oder verzweigten CON((C₁-C₆)-Alkyl)₂-, Cl-, Br-, I-, OH-, COCF₃- oder OSO₂CF₃-Gruppe; eine Benzyl-, Anisyl-, Pyridinylgruppe, eine Gruppe C(O)-W; C(S)-W oder C(NH)-W darstellen, worin
W eine lineare oder verzweigte (C₁-C₁₂)-Alkyl-, (C₃-C₁₂)-Cycloalkyl-, lineare oder verzweigte (C₁-C₁₂)-Alkoxy-, lineare oder verzweigte (C₁-C₁₂)-Alkylthiogruppe oder eine Gruppe NQQ1 darstellt, worin
Q und Q1 unabhängig voneinander ein Wasserstoffatom, eine lineare oder verzweigte (C₁-C₁₂)-Alkyl-, (C₃-C₁₂)-Cycloalkyl- oder CH(M)CO₂M1-Gruppe darstellt, worin
M und M1 unhabhängig voneinander ein Wasserstoffatom, eine lineare oder verzweigte (C₁-C₁₂)-Alkyl-, (C₃-C₁₂)-Cycloalkyl-, lineare oder verzweigte (C₂-C₁₂)-Alkenyl-, Phenyl-, Benzylgruppe, eine Gruppe CH₂-B oder CH₂-CH₂-B darstellen, worin B wie oben definiert ist,
mit Ausnahme der Verbindungen mit den folgenden Formeln: 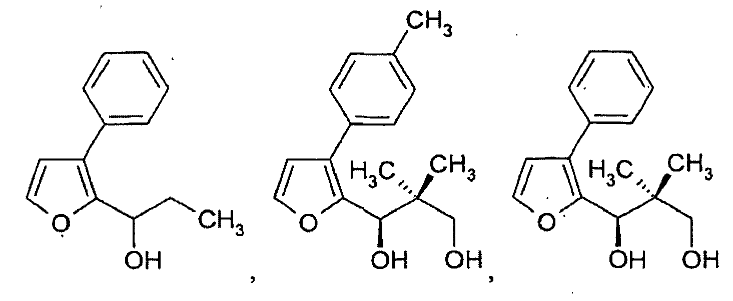 und

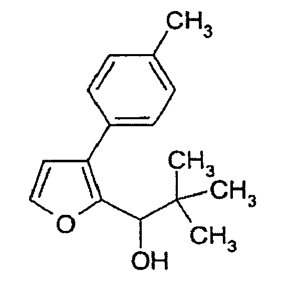

2. Verbindung nach Anspruch 1, in der Z für N steht.

3. Verbindung nach irgendeinem der vorangehenden Ansprüche, in der A die Gruppe CH darstellt.

4. Verbindung nach irgendeinem der vorangehenden Ansprüche, in der Y eine NH₂-Gruppe oder ein Wasserstoffatom darstellt.

5. Verbindung nach irgendeinem der vorangehenden Ansprüche, in der X eine lineare oder verzweigte (C₁-C₁₂)-Alkylgruppe, vorteilhaft eine Propylgruppe, darstellt.

6. Verbindung nach irgendeinem der vorangehenden Ansprüche, **dadurch gekennzeichnet, dass** sie ausgewählt ist aus der Gruppe bestehend aus 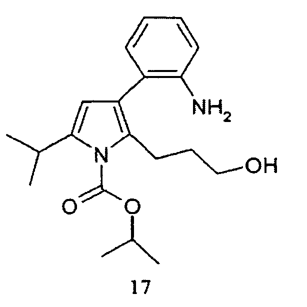 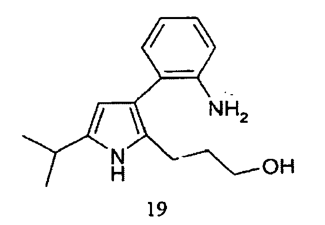 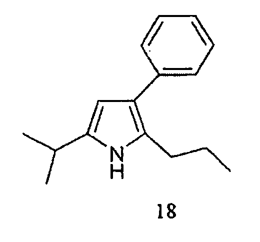 und

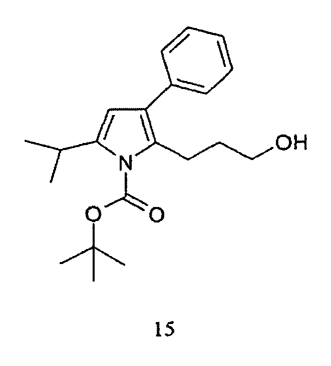

7. Verfahren zur Herstellung von Verbindungen nach irgendeinem der vorangehenden Ansprüche, umfassend die Schritte:
a) Synthese einer Pyrrolylalkylcarbinol-, Furanylalkylcarbinol- oder Thiophenylalkylcarbinol-Einheit der folgenden allgemeinen Formel II: 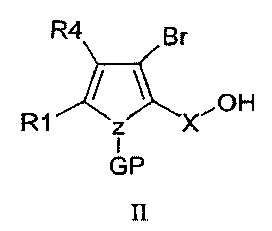 in der Z, X, R1 und R4 wie in Anspruch 1 definiert sind und GP eine Schutzgruppe des Stickstoffs darstellt, wenn Z für N steht, oder abwesend ist, wenn Z für O oder S steht,
b) Funktionalisierung der Pyrrolylalkylcarbinol-, Furanylalkylcarbinol- oder Thiophenylalkylcarbinol-Einheit, indem man eine Aryl- oder Heteroaryl-Einheit in die Position 3 des Pyrrol-, Furan- oder Thiophencyclus einführt.

8. Verfahren nach Anspruch 7 für den Fall, in dem X für (CH₂)₃ steht, **dadurch gekennzeichnet, dass** der Schritt a) aus der Cyclodehydratisierung von β-γ-ungesättigten Aminoketonen der folgenden Formel III besteht: 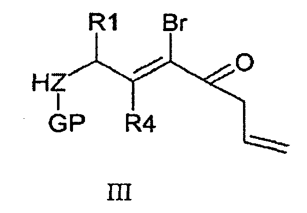 in der Z, R1, R4 und GP wie in Anspruch 7 definiert sind, um das Produkt der folgenden Formel IV zu erhalten 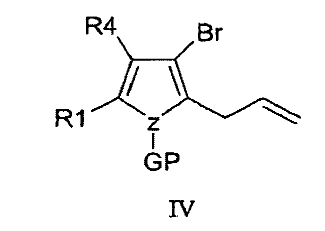 in der Z, R1, R4 und GP wie in Anspruch 7 definiert sind, gefolgt von der Einführung der Alkohol-Funktion durch Hydroborierung/Oxidation des Produkts der Formel IV, um das Produkt der Formel II wie in Anspruch 7 definiert zu erhalten.

9. Verfahren nach irgendeinem der Ansprüche 7 oder 8 für den Fall, in dem R2, R3, R4, R5 und R6 ein Wasserstoffatom darstellen, Y eine NH₂-Gruppe darstellt, A eine CH-Gruppe darstellt, X für (CH₂)₃ steht und Z für N steht, **dadurch gekennzeichnet, dass** der Schritt b) besteht aus:
- einer gemischten Palladium-katalysierten Kupplung vom Suzuki-Miyaura-Typ der Verbindung der Formel II, in der R4 ein Wasserstoffatom darstellt, X für (CH₂)₃ steht, Z für N steht und GP eine Schutzgruppe des Stickstoffs darstellt, und von 2-Triazenborsäure auf solche Weise, dass man eine Verbindung der folgenden Formel V erhält: 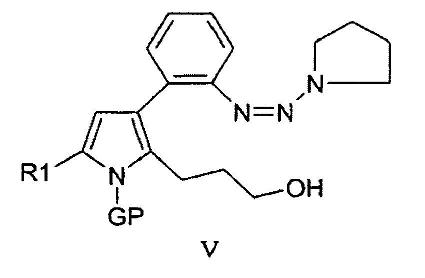 in der GP eine Schutzgruppe des Stickstoffs darstellt und R1 wie in Anspruch 1 definiert ist,
- einer Schutzgruppenentfernung von der Triazen-Funktion, um die Gruppe der folgenden Formel VI zu erhalten 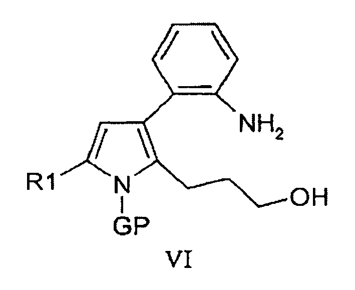 in der GP eine Schutzgruppe des Stickstoffs darstellt und R1 wie in Anspruch 1 definiert ist,
- einer spezifischen Schutzgruppenentfernung der Schutzgruppe GP, um die Verbindung der Formel I zu erhalten, in der R2, R3, R4, R5 und R6 ein Wasserstoffatom darstellen, Y eine NH₂-Gruppe darstellt, A eine CH-Gruppe darstellt, X für (CH₂)₃ steht, Z für N steht und R1 wie in Anspruch 1 definiert ist.

10. Pharmazeutische Zusammensetzung, umfassend eine Verbindung nach irgendeinem der Ansprüche 1 bis 6 oder eine Verbindung, die ausgewählt ist aus 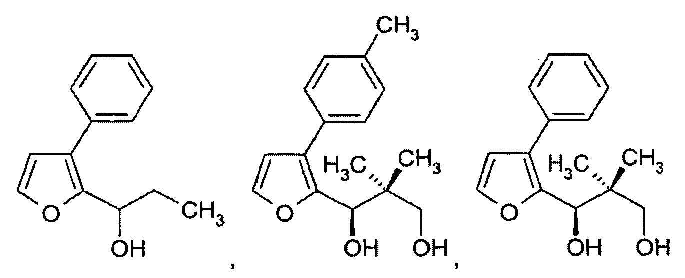 und 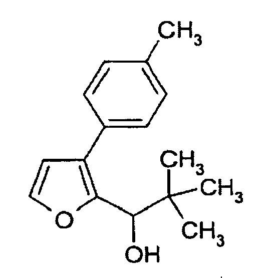 und einen pharmazeutisch annehmbaren Träger.

11. Verbindung nach irgendeinem der Ansprüche 1 bis 6 oder ausgewählt aus 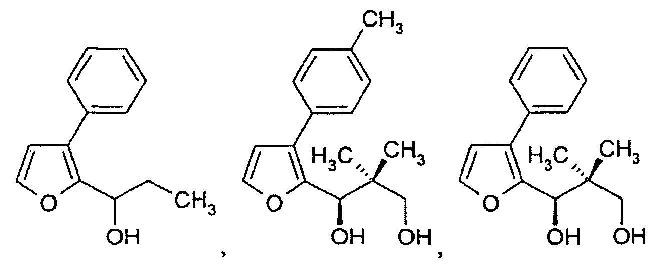 und 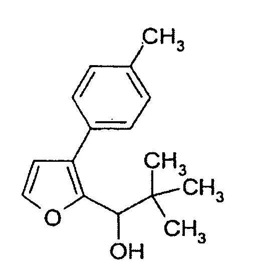 für ihre Verwendung als Medikament.

12. Verbindung nach Anspruch 11 als antimitotisches, vorteilhaft antitumorales Medikament.

13. Verbindung nach Anspruch 12 für ihre Verwendung als Medikament, das zur Behandlung oder Verhütung von Krebs bestimmt ist.

14. Verbindung der allgemeinen Formel V 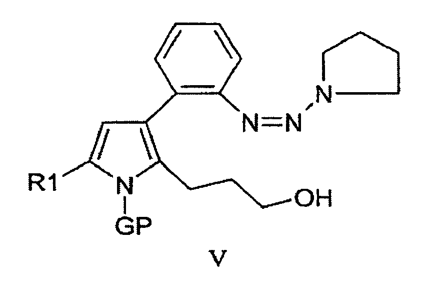 in der GP eine Schutzgruppe des Stickstoffs darstellt und R1 ein Wasserstoffatom, ein Fluoratom, eine lineare oder verzweigte (C₁-C₁₂)-Alkyl-, (C₃-C₁₂)-Cycloalkyl-, lineare oder verzweigte (C₂-C₁₂)-Alkenylgruppe, eine Gruppe CH₂-B oder CH₂-CH₂-B darstellt, worin B eine Phenylgruppe, die unsubstituiert oder durch ein oder mehrere Gruppen substituiert ist, die ausgewählt sind aus einer linearen oder verzweigten (C₁-C₆)-Alkyl-, linearen oder verzweigten (C₁-C₆)-Alkoxy-, NH₂-, NO₂-, CN-, COOH-, linearen oder verzweigten CO₂((C₁-C₆)-Alkyl)-, CONH₂-, linearen oder verzweigten CONH((C₁-C₆)-Alkyl)-, linearen oder verzweigten CON((C₁-C₆)-Alkyl)₂-, Cl-, Br-, I-, OH-, COCF₃-, OSO₂CF₃-Gruppe; eine Naphthyl-, Anthracenyl-, 9H-Fluorenylgruppe, die unsubstituiert ist oder an Position 9 durch eine oder zwei lineare oder verzweigte (C₁-C₁₂)-Alkylgruppen substituiert ist, Anisyl- oder Pyridinylgruppe darstellt.

15. Verbindung der allgemeinen Formel VI 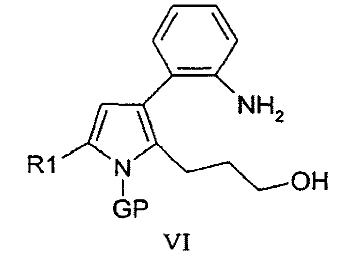 in der GP eine Schutzgruppe des Stickstoffs darstellt und R1 ein Wasserstoffatom, ein Fluoratom, eine lineare oder verzweigte (C₁-C₁₂)-Alkyl-, (C₃-C₁₂)-Cycloalkyl-, lineare oder verzweigte (C₂-C₁₂)-Alkenylgruppe, eine Gruppe CH₂-B oder CH₂-CH₂-B darstellt, worin B eine Phenylgruppe, die unsubstituiert ist oder durch eine oder mehrere Gruppen substituiert ist, die ausgewählt sind aus einer linearen oder verzweigten (C₁-C₆)-Alkyl-, linearen oder verzweigten (C₁-C₆)-Alkoxy-, NH₂-, NO₂-, CN-, COOH-, linearen oder verzweigten CO₂((C₁-C₆)-Alkyl)-, CONH₂-, linearen oder verzweigten CONH((C₁-C₆)-Alkyl)-, linearen oder verzweigten CON((C₁-C₆)-Alkyl)₂-, Cl-, Br-, I, OH-, COCF₃-, OSO₂CF₃-Gruppe; eine Naphthyl-, Anthracenyl-, 9H-Fluorenylgruppe, die unsubstituiert oder an Position 9 durch eine oder zwei lineare oder verzweigte (C₁-C₁₂)-Alkylgruppen substituiert ist, Anisyl- oder Pyridinylgruppe darstellt.
